(19) Europäisches Patentamt

European Patent Office

Office européen des brevets

(11) EP 1 618 897 A1

(12) EUROPEAN PATENT APPLICATION
published in accordance with Art. 158(3) EPC

(43) Date of publication:
25.01.2006 Bulletin 2006/04

(51) Int Cl.:
*A61K 47/42* (1990.01)   *A61K 47/48* (1990.01)
*A61K 31/704* (2000.01)   *A61K 9/127* (1990.01)
*A61K 9/107* (1990.01)   *A61K 9/16* (1974.07)
*A61K 9/19* (1995.01)   *A61K 39/395* (1985.01)
*A61P 35/00* (2000.01)   *A61P 35/04* (2000.01)

(21) Application number: 04725481.8

(22) Date of filing: 02.04.2004

(86) International application number:
PCT/JP2004/004876

(87) International publication number:
WO 2004/089419 (21.10.2004 Gazette 2004/43)

(84) Designated Contracting States:
AT BE BG CH CY CZ DE DK EE ES FI FR GB GR
HU IE IT LI LU MC NL PL PT RO SE SI SK TR
Designated Extension States:
AL HR LT LV MK

(30) Priority: 04.04.2003 JP 2003101521

(71) Applicants:
• The University of Tokyo
Tokyo 113-0033 (JP)
• Daiichi Fine Chemical Co., Ltd.
Takaoka-shi,
Toyama 933-8511 (JP)
• DAIICHI PHARMACEUTICAL CO., LTD.
Chuo-ku,
Tokyo 103-8234 (JP)

(72) Inventors:
• SEIKI, Motoharu,
5-203, Koyamadaijutaku
Tokyo 142-0061 (JP)
• YANA, Ikuo,
302, Yutaka Mansion
Kawasaki-shi,
Kanagawa 211-0051 (JP)

• AOKI, Takanori,
DAIICHI FINE CHEMICAL CO., LTD.
Takaoka-shi,
Toyama 933-8511 (JP)
• YASUDA, Junko,
DAIICHI FINE CHEMICAL CO., LTD.
Takaoka-shi,
Toyama 933-8511 (JP)
• KIKUCHI, H.,
DAIICHI PHARM. CO. LTD.
Edogawa-ku,
Tokyo 134-8630 (JP)
• ISHIDA, E.,
DAIICHI PHARMACEUTICAL CO. LTD.
Edogawa-ku,
Tokyo 134-8630 (JP)

(74) Representative: Sternagel, Fleischer, Godemeyer
& Partner
Patentanwälte,
An den Gärten 7
51491 Overath (DE)

(54) LIPID MEMBRANE STRUCTURE CONTAINING ANTI-MT-MMP MONOCLONAL ANTIBODY

(57) A lipid membrane structure containing an anti-membrane-type matrix metalloproteinase monoclonal antibody such as an anti-MT1-MMP monoclonal antibody as a component of the lipid membrane structure. Said structure can be utilized as a drug delivery system for efficiently delivering a medicinally active ingredient and/or a gene to tumor cells, neoplastic vessel and the like in which a membrane-type matrix metalloproteinase (MT-MMP) is expressed.

**Description**

Technical Field

The present invention relates to a novel lipid membrane structure containing an anti-membrane-type matrix metalloproteinase monoclonal antibody.

Background Art

Matrix metalloproteinases (MMPs) constitute a family of zinc-dependent endopeptidases which degrade various constitutive proteins of the extracellular matrix (ECM) and basal membrane components and are considered essential for extracellular matrix metabolism. It has been elucidated that the class of enzymes relate to reconstruction of connective tissues such as development of normal germs, bone growth, and wound healing and are also involved in various kinds of pathological processes such as those of atherosclerosis, pulmonary emphysema, rheumatoid arthritis, and infiltration and metastasis of cancer. To date, many mammalian MMPs have been analyzed to an amino acid level by cDNA cloning.

As the mammalian MMPs, for example, MMP-1 (collagenase), MMP-2 (gelatinase A), MMP-3 (stromelysin-1), MMP-7 (matrilysin), MMP-8 (neutrophil collagenase), MMP-9 (gelatinase B), MMP-10 (stromelysin-2), MMP-11 (stromelysin-3), MMP-12 (macrophage elastase), MMP-13 (collagenase-3), MMP-14 (MT1-MMP), MMP-15 (MT2-MMP), MMP-16 (MT3-MMP), MMP-17 (MT4-MMP), MMP-19, MMP-20 (enamelysin), MMP-24 (MT5-MMP), MMP-25 (MT6-MMP) and the like are known. These MMPs are classified into at least 4 kinds of subfamilies, i.e., collagenases, gelatinases, stromelysins, and membrane-type matrix metalloproteinases (MT-MMPs), on the basis of primary structures, substrate specificity, and cell distribution. Among them, the MT-MMP subfamily was reported latest as a subclass of MMPs, and MT1-MMP, MT2-MMP, MT3-MMP, MT4-MMP, MT5-MMP, MT6-MMP and the like have so far been isolated and identified by using degenerate primers for regions conserved among MMPs and RT-PCR (Sato, H. et al., Nature, 370, 61-65 (1994); Will, H. et al., Eur. J. Biochem., 231, 602-608 (1995); Takino, T. et al., J. Biol. Chem., 270, 23013-23020 (1995); Puente X.S. et al., Cancer Res., 56, 944-949 (1996); Japanese Patent Unexamined Publication (KOKAI) No. 2000-270874; Pei, D., J. Biol. Chem., 274, 8925-8932 (1999); Kajita, M. et al., FEBS Lett., 457, 353-356 (1999)).

MT-MMPs are type I membrane proteins which have a single transmembrane domain and a short cytoplasmic tail following the hemopexin domain unique to many MMPs. Further, an insertion of basic amino acids is commonly present in these proteins between the propeptide and the active domain. Cleavage by furin or a furin-like enzyme induces the activation of these membrane proteins (Pei, D., and Weiss, S. J., J. Biol. Chem., 271, 9135-9140 (1996); Sato H. et al., FEBS Lett., 393, 101-104 (1996); Cao, J. et al., J. Biol. Chem., 271, 30174-30180, 1996).

When cells migrate, invade, and metastasize in a tissue, the step of degradation of extracellular matrices (ECMs) surrounding the cells is essential. Those playing a key role in that step are the class of enzymes called MMPs. Among them, a role of MT1-MMP expressed on a cell membrane surface is considered important for migration, invasion, metastasis, and angiogenesis of cancers. MT1-MMP is an enzyme also called as membrane-type 1 matrix metalloproteinase (MT1-MMP) or MMP-14 (MEROPS ID: M10.014), and reported as a product of a gene that occupies chromosomal locus 14q11-q12 in a human (Migon, C. et al., Genomics, 28, 360-361 (1995)). Existence of this enzyme has been confirmed and detailed structure and properties thereof have been elucidated by DNA cloning and expression of recombinant proteins (Sato, H. et al., Nature, 370, 61-65 (1994); Takino, T., et al., Gene, 155, 293-298 (1995); Japanese Unexamined Patent Publication No. 7-203961, 7-303482; GenBank.TM. accession number: D26512). The presence of MT1-MMP has been also confirmed in dogs, goats, rabbits, wild boars, mice and the like, as well as humans. The cDNA of human MT1-MMP encodes 582 amino acid residues (EMBL accession No. D26512, E09720 and E 10297, SWISS-PROT: P50281), of which structure is composed of a signal peptide followed by a propeptide domain, an insertion sequence composed of 10 specific amino acid residues similar to stromelysin-3 (a potential sequence for a furin-like enzyme recognition site), a core enzyme domain having a potential site as a zinc binding site, a hinge domain, and a hemopexin-like domain, and a transmembrane (TM) domain.

It has been elucidated so far that MT1-MMP activates a potential type of gelatinase A (progelatinase A/72 kDa type IV collagenase, proMMP-2), which is also an MMP member and a basal membrane decomposing enzyme, and further MT1-MMP per se also degrades various ECM molecules such as collagen type I, II and III, fibronectin, laminin, vitronectin, and aggrecan. In addition, it has also been demonstrated that MT1-MMP promotes tumor invasion and metastasis processes (Seiki, M., Apmis, 107, 137-143 (1999); Sato, H., et al., Nature, 370, 61-65 (1994)). Furthermore, it is also known that MT1-MMP activates other MMPs such as proMMP-2 (Sato, H., et al., Nature, 370, 61-65 (1994)) and procollagenase-3 (proMMP-13) (Knauper, V., et al., J. Biol. Chem., 271, 17124-17131 (1996)). As described above, the expression of MT1-MMP may be involved in the initiation of variety of proteinase cascades on cell surfaces, and it has also been shown that MT1-MMP is involved in not only invasion and metastasis of cancer cells (Seiki, M., Apmis, 107, 137-143 (1999); Sato, H., et al., Nature, 370, 61-65 (1994)), but also in other physiological processes such as those of angiogenesis (Hiraoka, N., et al., Cell, 95, 365-377 (1998); Zhou, Z., et al., Proc. Natl. Acad. Sci. USA, 97, 4052-4057 (2000)) and skeletal development (Zhou, Z., et al., Proc. Natl. Acad. Sci. USA, 97, 4052-4057 (2000); Holmbeck, K., et al., Cell, 99, 81-92 (1999)). Thus, MT1-MMP is considered to be a tool necessary for physiological and pathological cellular invasion in tissues.

Various lipid membrane structures containing monoclonal antibodies have so far been proposed as drug delivery systems. However, a lipid membrane structure having fully satisfactory performance has not yet been provided. Further, a lipid membrane structure containing an anti-membrane-type matrix metalloproteinase monoclonal antibody has not yet been known to date.

Disclosure of the Invention

An object of the present invention is to provide a lipid membrane structure containing an anti-membrane-type matrix metalloproteinase monoclonal antibody (hereinafter in the specification, membrane-type matrix metalloproteinase may be abbreviated as "MT-MMP", and anti-membrane-type matrix metalloproteinase monoclonal antibody may be abbreviated as "anti-MT-MMP monoclonal antibody"). More specifically, the object of the present invention is to provide a lipid membrane structure containing an anti-MT-MMP monoclonal antibody as a drug delivery system for efficiently delivering a medicinally active ingredient and/or a gene to a tumor cell or the like in which MT-MMP is expressed.

The inventors of the present invention conducted various researches to achieve the aforementioned object, and as a result, they succeeded in providing a lipid membrane structure containing an anti-MT-MMP monoclonal antibody, and found that this lipid membrane structure successfully delivered a medicinally active ingredient and/or a gene efficiently to tumor cells in which MT-MMP was expressed. The inventors of the present invention also found that the aforementioned lipid membrane structure successfully delivered a medicinally active ingredient and/or a gene also efficiently to an angiogenesis front in the inside of tumor. Specifically, the lipid membrane structure of the present invention can simultaneously target tumor cells and neoplastic vessels, in which MT-MMP is expressed, and can deliver a medicinally active ingredient and/or a gene efficiently to both of them. Conventional lipid membrane structures target either tumor cells or neoplastic vessels. Thus, the lipid membrane structure that can simultaneously target both of tumor cells and neoplastic vessels was first achieved by the present invention. By applying conventional techniques, only a solid tumor grown to some extent can be targeted. In contrast, by the lipid membrane structure of the present invention, a medicinally active ingredient and/or a gene can be delivered to a tumor tissue even in a small stage in which generation of neoplastic vessels is being started, thereby a therapeutic treatment can be attained. The present invention was achieved on the basis of these findings.

The present invention thus provides a lipid membrane structure containing an anti-membrane-type matrix metalloproteinase monoclonal antibody. According to preferred embodiments of the above invention, there are provided the aforementioned lipid membrane structure, wherein the monoclonal antibody is present in a lipid membrane, on a surface of lipid membrane, in an internal space of lipid membrane, in a lipid layer, and/or on a surface of lipid layer of the lipid membrane structure; the aforementioned lipid membrane structure, which comprises the monoclonal antibody as a component of the lipid membrane structure; and the aforementioned lipid membrane structure, wherein the monoclonal antibody binds to a membrane surface of the lipid membrane structure.

According to more preferred embodiments, there are provided the aforementioned lipid membrane structure, wherein the monoclonal antibody consists of one or more kinds of monoclonal antibodies selected from an anti-MT1-MMP monoclonal antibody, an anti-MT2-MMP monoclonal antibody, an anti-MT3-MMP monoclonal antibody, an anti-MT4-MMP monoclonal antibody, an anti-MT5-MMP monoclonal antibody and an anti-MT6-MMP monoclonal antibody; the aforementioned lipid membrane structure, wherein the monoclonal antibody is a human monoclonal antibody or a mouse monoclonal antibody; the aforementioned lipid membrane structure, wherein the monoclonal antibody is a Fab fragment, a $F(ab')_2$ fragment, or a Fab' fragment; the aforementioned lipid membrane structure, which contains a substance for binding the monoclonal antibody to the lipid membrane structure; and the aforementioned lipid membrane structure, wherein the substance for binding the monoclonal antibody to the lipid membrane structure is a lipid derivative that can react with mercapto group in the anti-MT-MMP monoclonal antibody or a fragment thereof.

The present invention also provides the aforementioned lipid membrane structure, which contains a phospholipid and/or a phospholipid derivative as a component of the lipid membrane structure; the aforementioned lipid membrane structure, wherein the phospholipid and/or the phospholipid derivative consists of one or more kinds of phospholipids and/or phospholipid derivatives selected from the group consisting of phosphatidylethanolamine, phosphatidylcholine, phosphatidylserine, phosphatidylinositol, phosphatidylglycerol, cardiolipin, sphingomyelin, ceramide phosphorylethanolamine, ceramide phosphorylglycerol, ceramide phosphorylglycerol phosphate, 1,2-dimyristoyl-1,2-deoxyphosphatidylcholine, plasmalogen, and phosphatidic acid; the aforementioned lipid membrane structure, which further contains a sterol as a component of the lipid membrane structure; and the aforementioned lipid membrane structure, wherein the sterol is cholesterol and/or cholestanol.

The present invention further provides the aforementioned lipid membrane structure, which has a blood retentive function; the aforementioned lipid membrane structure, which contains a blood retentive lipid derivative as a component of the lipid membrane structure; the aforementioned lipid membrane structure, wherein the blood retentive lipid derivative is a polyethylene glycol-lipid derivative or a polyglycerin-phospholipid derivative; the aforementioned lipid membrane structure, wherein the polyethylene glycol-lipid derivative consists of one or more kinds of polyethylene glycol-lipid derivatives selected from the group consisting of N-{carbonyl-methoxypolyethylene glycol-2000}-1,2-dipalmitoyl-sn-glycero-3-phosphoethanolamine, N-{carbonyl-methoxypolyethylene glycol-5000}-1,2-dipalmitoyl-sn-glycero-3-phosphoethanolamine, N-{carbonyl-methoxypolyethylene glycol-750}-1,2-distearoyl-sn-glycero-3-phosphoethanolamine, N-{carbonyl-methoxypolyethylene glycol-2000}-1,2-distearoyl-sn -glycero-3-phosphoethanolamine and N-{carbonyl-methoxypolyethylene glycol-5000}-1,2-distearoyl-sn-glycero-3-phosphoethanolamine; the aforementioned lipid membrane structure, which has a function of sensitivity to a change of temperature; the aforementioned lipid membrane structure, which contains a temperature-sensitive lipid derivative as a component in the lipid membrane structure; the aforementioned lipid membrane structure, wherein the temperature-sensitive lipid derivative is dipalmitoylphosphatidylcholine; the aforementioned lipid membrane structure, which has a pH-sensitive function; the aforementioned lipid membrane structure, which contains a pH-sensitive lipid derivative as a component of the lipid membrane structure; and the aforementioned lipid membrane structure, wherein the pH-sensitive lipid derivative is dioleoylphosphatidylethanolamine.

The present invention also provides the aforementioned lipid membrane structure, which reacts with a membrane-type matrix metalloproteinase on a tumor cell membrane; the aforementioned lipid membrane structure, wherein the tumor cell is an MT-MMP expressing cell; the aforementioned lipid membrane structure, wherein the tumor cell is a cell of fibrosarcoma, squamous carcinoma, neuroblastoma, breast carcinoma, gastric cancer, hepatoma, bladder cancer, thyroid tumor, urinary tract epithelial cancer, glioblastoma, acute myeloid leukemia, pancreatic duct cancer, or prostate cancer; the aforementioned lipid membrane structure, which reacts with a membrane-type matrix metalloproteinase of a neoplastic vessel; the aforementioned lipid membrane structure, wherein the lipid membrane structure is in the form of micelle, emulsion, liposome, or a mixture thereof; the aforementioned lipid membrane structure, which is in a form of dispersion in an aqueous solvent, a lyophilized form, a spray-dried form, or a frozen form.

From another aspect, the present invention provides a pharmaceutical composition containing the aforementioned lipid membrane structure and a medicinally active ingredient and/or a gene. According to preferred embodiments of this invention, there are provided the aforementioned pharmaceutical composition, wherein the medicinally active ingredient and/or gene exists in a lipid membrane, on a surface of lipid membrane, in an internal space of lipid membrane, in a lipid layer, and/or on a surface of lipid layer of the lipid membrane structure; and the aforementioned pharmaceutical composition, which is in a form of dispersion in an aqueous solvent, a lyophilized form, a spray-dried form, or a frozen form.

The present invention provides a method for estimating an amount of anti-membrane-type matrix metalloproteinase monoclonal antibody contained in the aforementioned lipid membrane structure, wherein a competitive reaction with an antigenic substance caused by both of an enzyme-labeled monoclonal antibody, prepared from an anti-membrane-type matrix metalloproteinase monoclonal antibody by a known method, and the lipid membrane structure is detected by an enzyme immunoassay technique.

From further aspects, the present invention provides a method for prophylactic and/or therapeutic treatment of various MT-MMP-related diseases such as tumor, which comprises the step of administering a pharmaceutical composition comprising the aforementioned lipid membrane structure and a medicinally active ingredient and/or a gene to a mammal including human; and a method for delivering a medicinally active ingredient and/or a gene to a tumor cell and/or a neoplastic vessel, which comprises the step of administering a medicinally active ingredient and/or a gene in a state of being retained by the aforementioned lipid membrane structure to a mammal including human.

Brief Explanation of the Drawings

Fig. 1 shows results of affinity purification of IgG from ascites containing anti-MT1-MMP monoclonal antibodies (IgG) using a recombinant protein A Sepharose FF gel column.

Fig. 2 shows results of gel filtration of the purified IgG after digestion with pepsin.

Fig. 3 shows results of gel filtration of a F(ab')$_2$ fraction after a reduction treatment.

Fig. 4 shows results of gel filtration of a product obtained by mixing a Fab' fraction and maleinimide group-introduced and anticancer agent (DOX)-encapsulating liposomes at a maleinimide molar ratio of 1:1 and allowing the mixture to react for 20 hours at a low temperature and under light shielding.

Fig. 5 shows results of gel filtration of a product obtained by mixing a Fab' fraction and maleinimide group-introduced and anticancer agent (DOX)-encapsulating liposomes at a maleinimide molar ratio of 1:3 and allowing the mixture to react for 20 hours at a low temperature and under light shielding.

Fig. 6 is a photograph showing reduced SDS-PAGE patterns of anti-MT1-MMP monoclonal antibody-binding liposomes and maleinimide group-introduced liposomes. The positions of bands for a size expected to be that of Fab' binding to the liposomes are indicated with arrows. Lanes 1, 3 and 5 indicate the results for Fab'-DOX-LP (Preparation Examples (10), ② and ③), Lanes 2, 4 and 6 indicate the results for Fab'-LP (Preparation Examples ⑨, ⑥ and ⑦), Lane 7 indicates the result for LP-mal, Lane 8 indicates the result for DOX-LP-mal, and M indicates a molecular weight marker.

Fig. 7 shows cytostatic effect of each of the liposomes. The left half of the drawing represents the results obtained by using HT1080 cells, which are MT1-MMP-expressing cells, and the right half of the drawing represents the results obtained by using MCF-7 cells, which do not express MT1-MMP. In the table on the right side, cytostatic rates of the test groups, relative to the number of cells in the control group proliferated during culture of 24 hours from the start (immediately after washing of the cells), are shown.

Fig. 8 shows results of cytostatic test. The dose-dependency of the cytostatic effect of Fab'-DOX-LP was demonstrated.

Fig. 9 shows a schematic view of the cell adhesion test in a mouse peritoneum inoculation (HT1080) model. Appearance of peritoneal tumors of models intraperitoneally administered with LP (upper drawing) or Fab'-LP (lower drawing) are schematically indicated. The portions indicated with □, i.e., cleaved faces of one part from the inside of the tumor and 2 parts from the tumor surface layer, were photographed.

Fig. 10 is a photograph showing reduced SDS-PAGE patterns of anti-MT1-MMP monoclonal antibody (clone number: 222-2D12)-binding liposomes and F(ab')$_2$ (clone number: 222-2D12). The positions of bands corresponding to a size expected to be that of Fab' binding to the liposomes are indicated with arrows.

Fig. 11 shows results of the cytostatic test using the HT1080 cells. The numerical values mentioned in the notes represent doxorubicin concentrations in the specimens.

Fig. 12 shows results of the cytostatic test in the same manner as Fig. 11.

Best Mode for Carrying out the Invention

The lipid membrane structure of the present invention is characterized to contain an anti-MT-MMP monoclonal antibody. As components other than the anti-MT-MMP monoclonal antibody, the lipid membrane structure of the present invention contains membrane components constituting the lipid membrane structure. As the aforementioned membrane component, for example, a phospholipid and/or a phospholipid derivative is preferably used. Examples of the phospholipid and phospholipid derivative include, for example, phosphatidylethanolamine, phosphatidylcholine, phosphatidylserine, phosphatidylinositol, phosphatidylglycerol, cardiolipin, sphingomyelin, ceramide phosphorylethanolamine, ceramide phosphorylglycerol, ceramide phosphorylglycerol phosphate, 1,2-dimyristoyl-1,2-deoxyphosphatidylcholine, plasmalogen, phosphatidic acid and the like, and these may be used alone or two or more kind of them can be used in combination. The fatty acid residues of these phospholipids are not particularly limited, and examples thereof include a saturated or unsaturated fatty acid residue having 12 to 20 carbon atoms. Specific examples include an acyl group derived from a fatty acid such as lauric acid, myristic acid, palmitic acid, stearic acid, oleic acid and linoleic acid. Further, phospholipids derived from natural products such as egg yolk lecithin and soybean lecithin can also be used.

The lipid membrane structure of the present invention may further contain, as a membrane component other than the phospholipid and/or phospholipid derivative, a sterol such as cholesterol, and cholestanol, a fatty acid having a saturated or unsaturated acyl group having 8 to 22 carbon atoms and an antioxidant such as α- tocopherol. However, the membrane component is not limited to these examples.

To the lipid membrane structure of the present invention, one or more functions can be imparted such as, for example, blood retentive function, temperature change-sensitive function, pH-sensitive function and the like, and by imparting one or more of these functions, for example, residence in blood of the pharmaceutical composition of the present invention consisting of the lipid membrane structure containing a medicinally active ingredient and/or a gene can be improved, a rate of capture by reticuloendothelial systems of liver, spleen and the like can be reduced, or a releasing property of medicinally active ingredient and/or gene can be enhanced.

Examples of blood retentive lipid derivatives which can impart the blood retentive function include, for example, glycophorin, ganglioside GM1, phosphatidylinositol, ganglioside GM3, glucuronic acid derivative, glutamic acid derivative, polyglycerin-phospholipid derivative, polyethylene glycol derivatives such as N-{carbonyl-methoxypolyethylene glycol-2000}-1,2-dipalmitoyl-sn-glycero-3-phosphoethanolamine, N-{carbonyl-methoxypolyethylene glycol-5000}-1,2-dipalmitoyl-sn-glycero-3-phosphoethanolamine, N-{carbonyl-methoxypolyethylene glycol-750}-1,2-distearoyl-sn-glycero-3-phosphoethanolamine, N-{carbonyl-methoxypolyethylene glycol-2000}-1,2-distearoyl-sn-glycero-3-phosphoethanolamine and N-{carbonyl-methoxypolyethylene glycol-5000}-1,2-distearoyl-sn-glycero-3-phosphoethanolamine, and the like.

Examples of temperature change-sensitive lipid derivatives that can impart the temperature change-sensitive function include, for example, dipalmitoylphosphatidylcholine and the like. Examples of pH-sensitive lipid derivatives that can impart the pH-sensitive function include, for example, dioleoylphosphatidylethanolamine and the like.

Although the form of the lipid membrane structure of the present invention is not particularly limited, for example, a form in which the anti-MT-MMP monoclonal antibody forms the lipid membrane structure together with the phospholipid as a membrane component of the lipid membrane structure is preferred. More specifically, examples include, for example, a form in which the anti-MT-MMP monoclonal antibody exists (binds) at one or more kinds of positions selected from the group consisting of positions in the lipid membrane, on the lipid membrane surface of the lipid membrane structure, in an internal space of the lipid membrane structure, in a lipid layer, and on a lipid layer surface. More preferred examples include a form in which the anti-MT-MMP monoclonal antibody serves as a membrane component together with the phospholipid and the like to form the lipid membrane structure, and a form in which the anti-MT-MMP monoclonal antibody binds to the lipid membrane surface of the lipid membrane structure.

The form and production method of the lipid membrane structure of the present invention are not particularly limited. Examples of the form include a dry mixture form, a form in which the lipid membrane structure is dispersed in an aqueous solvent, a form obtained by drying or freezing any of the forms mentioned above and the like. The methods for producing the lipid membrane structures of these forms will be explained below. However, the form of the lipid membrane structure of the present invention and the methods for preparing thereof are not limited to the aforementioned forms and the production methods explained below.

(1) Production method using all the components of lipid membrane structure

The lipid membrane structure in the form of dried mixture can be produced by, for example, once dissolving all the components of the lipid membrane structure in an organic solvent such as chloroform and then subjecting the resulting solution to solidification under reduced pressure by using an evaporator or spray drying by using a spray dryer.

The form of the lipid membrane structure dispersed in an aqueous solvent can be prepared by adding the aforementioned dried mixture to an aqueous solvent and emulsifying the mixture by using an emulsifier such as a homogenizer, ultrasonic emulsifier, high pressure jet emulsifier or the like. Further, the aforementioned form can also be prepared by a method known as a method for preparing liposomes, for example, the reverse phase evaporation method or the like. When it is desired to control a size of the lipid membrane structure, extrusion can be performed under high pressure by using a membrane filter of uniform pore sizes or the like. Examples of the form in which lipid membrane structures are dispersed in an aqueous solvent include unilamella liposomes, multi-lamella liposomes, O/W type emulsions, W/O/W type emulsions, spherical micelles, fibrous micelles, layered structures of irregular shapes and the like. An example of preferred forms of the lipid membrane structure of the present invention includes liposomes. The size of the lipid membrane structure in the dispersed state should not be particularly limited. For example, the particle diameter of liposomes or particles in emulsion is 50 nm to 5 $\mu$ m, preferably 50 nm to 400 nm, more preferably 50 nm to 200 nm, still more preferably 50 nm to 150 nm. The particle diameter of spherical micelle is 5 to 100 nm. Where a fibrous micelle or irregular layered structure is prepared, the thickness of one layer thereof is 5 to 10 nm, and such layers form a single layer. The particle diameter means a weight average particle diameter determined by the quasi-elastic light scattering method.

The composition of the aqueous solvent (dispersion medium) should not be particularly limited, and examples include, for example, a buffer such as phosphate buffer, citrate buffer, and phosphate-buffered physiological saline, physiological saline, a medium for cell culture and the like. Although the lipid membrane structure can be stably dispersed in these aqueous solvents (dispersion media), the solvents may be further added with a saccharide (aqueous solution), for example, a monosaccharide such as glucose, galactose, mannose, fructose, inositol, ribose and xylose, disaccharide such as lactose, sucrose, cellobiose, trehalose and maltose, trisaccharide such as raffinose and melezitose, and polysaccharide such as cyclodextrin, sugar alcohol such as erythritol, xylitol, sorbitol, mannitol, and maltitol, or a polyhydric alcohol (aqueous solution) such as glycerin, diglycerin, polyglycerin, propylene glycol, polypropylene glycol, ethylene glycol, diethylene glycol, triethylene glycol, polyethylene glycol, ethylene glycol mono-alkyl ether, diethylene glycol mono-alkyl ether and 1,3-butylene grycol. In order to stably store the lipid membrane structure dispersed in such an aqueous solvent (dispersion medium) for a long period of time, it is desirable to minimize electrolytes in the aqueous solvent (dispersion medium) from a viewpoint of physical stability such as prevention of aggregation. Further, from a viewpoint of chemical stability of lipids, it is desirable to control pH of the aqueous solvent (dispersion medium) to be in a range of from weakly acidic pH to around neutral pH (pH 3.0 to 8.0), and to remove dissolved oxygen by nitrogen bubbling.

Further, the dried or frozen form of the form in which the lipid membrane structure is dispersed in an aqueous solvent can be produced by drying or freezing the aforementioned lipid membrane structure dispersed in an aqueous solvent by an ordinary drying or freezing method based on lyophilization or spray drying. When a lipid membrane structure dispersed in the aqueous solvent is first prepared and then successively dried, it becomes possible to store the lipid membrane structure for a long period of time. In addition, when an aqueous solution containing a medicinally active ingredient is added to the dried lipid membrane structure, the lipid mixture is efficiently hydrated and thereby the medicinally active ingredient can be efficiently retained in the lipid membrane structure, which provides an advantageous effect.

When lyophilization or spray drying is carried out, a use of a saccharide (as an aqueous solution), for example, a monosaccharide such as glucose, galactose, mannose, fructose, inositol, ribose and xylose, disaccharide such as lactose, sucrose, cellobiose, trehalose and maltose, trisaccharide such as raffinose and melezitose, and polysaccharide such as cyclodextrin, or a sugar alcohol such as erythritol, xylitol, sorbitol, mannitol, and maltitol may achieve stable storage of the lipid membrane structure for a long period of time. For the freezing, a use of the aforementioned saccharide (as an aqueous solution) or a polyhydric alcohol (aqueous solution) such as glycerin, diglycerin, polyglycerin, propylene glycol, polypropylene glycol, ethylene glycol, diethylene glycol, triethylene glycol, polyethylene glycol, ethylene glycol mono-alkyl ether, diethylene glycol mono-alkyl ether and 1,3-butylene glycol may achieve stable storage of the lipid membrane structure for a long period of time. A saccharide and a polyhydric alcohol may be used in combination. A concentration of the saccharide or polyhydric alcohol in the form in which the lipid membrane structure is dispersed in an aqueous solvent is not particularly limited. In a state that the lipid membrane structure is dispersed in an aqueous solvent, for example, the concentration of the saccharide is preferably 2 to 20% (W/V), more preferably 5 to 10% (W/V), and the concentration of the polyhydric alcohol is preferably 1 to 5% (W/V), more preferably 2 to 2.5% (W/V). When a buffer is used as the aqueous solvent (dispersion medium), a concentration of the buffering agent is preferably 5 to 50 mM, more preferably 10 to 20 mM. The concentration of the lipid membrane structure in an aqueous solvent (dispersion medium) should not be particularly limited. However, the concentration of the total amount of lipids in the lipid membrane structure is preferably 0.1 to 500 mM, more preferably 1 to 100 mM.

(2) Stepwise production method (method of preparing the lipid membrane structure by using a part or all of the components other than the anti-MT-MMP monoclonal antibody and then binding the anti-MT-MMP monoclonal antibody to a membrane surface of the lipid membrane structure)

The lipid membrane structure in the form of dried mixture can be produced by first dissolving a part or all of the components of the lipid membrane structure other than the anti-MT-MMP monoclonal antibody in an organic solvent such as chloroform, and then adding the anti-MT-MMP monoclonal antibody and remaining components of the lipid membrane structure if desired, followed by subjecting the resulting mixture to solidification under reduced pressure by using an evaporator or spray drying by using a spray dryer.

The form of the lipid membrane structure dispersed in an aqueous solvent can be prepared by adding the aforementioned dried mixture comprising a part or all of the components other than the anti-MT-MMP monoclonal antibody to an aqueous solvent, emulsifying the mixture by using an emulsifier such as a homogenizer, ultrasonic emulsifier, high pressure jet emulsifier or the like, and then adding the anti-MT-MMP monoclonal antibody and the remaining components of the lipid membrane structure if desired. Further, the aforementioned form can also be prepared by a method known as a method for preparing liposomes, for example, the reverse phase evaporation method, instead of the emulsification. The resulting lipid membrane structure in the form in which the lipid membrane structure is dispersed in an aqueous solvent can be dried (lyophilization and spray drying) or frozen by an ordinary method.

In the present invention, the lipid membrane structure containing an anti-MT-MMP monoclonal antibody prepared by the production method mentioned in (2) above is preferred from a viewpoint of efficiency of delivery of a medicinally active ingredient and/or a gene. Examples of the method of allowing the anti-MT-MMP monoclonal antibody to be present on or to bind to the surface of the membrane of the lipid membrane structure include a known method (STEALTH LIPOSOME, pp.233-244, published by CRC Press, Inc., Edited by Danilo Lasic and Frank Martin) or similar methods. For example, as a component of the lipid membrane structure, a lipid derivative may be added that can react with mercapto group in the anti-MT-MMP monoclonal antibody (e.g., Fab fragment, F(ab')$_2$ fragment, Fab' fragment and the like), specifically, a lipid derivative having a maleinimide structure such as poly(ethylene glycol)- $\alpha$ -distearoylphosphatidylethanolamine- $\omega$ -maleinimide and $\alpha$ - [N-(1,2-distearoyl-sn-glycero-3-phosphorylethyl)carbamyl]- $\omega$ -{3-[2-(2,5-dihydro-2,5-dioxo-1H-pyrrol-1-yl)ethanecarboxamido]propyl}-poly(oxy-1,2-ethanediyl), thereby the anti-MT-MMP monoclonal antibody can be allowed to be present on or to bind to the surface of the membrane of the lipid membrane structure.

The anti-MT-MMP antibody may consist of a single kind of monoclonal antibody that can recognize a desired extracellular domain of MT-MMP and/or a related peptide fragment and the like, or a composition comprising two or more kinds of monoclonal antibodies having specificity for various epitopes. Moreover, the antibody may be a monovalent antibody or a multivalent antibody, and an naturally occurring type (intact) molecule, or a fragment or derivative thereof may be used. For example, a fragment such as F(ab')$_2$, Fab' and Fab may be used, and a chimeric antibody or hybrid antibody having at least two of antigen- or epitope-binding sites, a double specificity recombinant antibody such as quadrome and triome, an interspecies hybrid antibody, an anti-idiotype antibody and a chemically modified or processed version of these considered as a derivative of any of the foregoing antibodies may also be used. Further, those may be used include, for example, an antibody obtained by a synthetic or semisynthetic technique with applying a known cell fusion or hybridoma technique or a known antibody engineering technique, an antibody prepared by using a DNA recombinant technique by applying a conventional technique known from a viewpoint of antibody production, and an antibody having a neutralization or binding property for MT-MMP or a target epitope.

A monoclonal antibody specifically recognizing MT-MMP can be produced by an arbitrary method. The term "monoclonal" means being a population of substantially homogeneous antibodies, and the term should not be construed in any limitative way that the antibody should be produced by a certain specific method. Although each monoclonal antibody may contain a trace amount of a mutant that naturally occurs, each antibody consists of a population of substantially identical antibodies. As described above, the monoclonal antibody used in the present invention includes a hybrid antibody and a recombinant antibody, and regardless of an origin and a classification from viewpoints of immunoglobulin class and subclass thereof, a domain of a variable region may be replaced with a domain of a constant region (e.g., a humanized antibody), a light chain may be replaced with a heavy chain, a chain from a certain species may be replaced with a chain from another species, or the antibody may be fused with a heterogeneous protein, so long as the antibody has a desired biological activity. A modified monoclonal antibody mentioned above can also be used for the present invention. Techniques for these modifications are described in, for example, U.S. Patent No. 4,816,567; Monoclonal Antibody Production Techniques and Applications, 79-97, Marcel Dekker, Inc., New York, 1987; Morrison et al., Proc. Natl. Acad. Sci. USA, 81, 6851-6855 (1984) and the like.

Examples of preferred methods for producing a monoclonal antibody include the hybridoma method (Kohler, G. and Milstein, C., Nature, 256, 495-497 (1975); Human B cell hybridoma method (Kozbor et al., Immunology Today, 4, 72-79 (1983); Kozbor, J. Immunol., 133, 3001 (1984); Brodeur et al., Monoclonal Antibody Production Techniques and Applications, 51-63, Marcel Dekker, Inc., New York (1987)); trioma method; and EBV-hybridoma method (Cole et al., Monoclonal Antibodies and Cancer Therapy, Alan R. Liss, Inc., 77-96 (1985) (method for producing a human monoclonal antibody); U.S. Patent No. 4,946,778 (technique for producing a single chain antibody)). As references concerning antibody, there can be mentioned Biocca, S. et al., EMBO J, 9, 101-108 (1990); Bird, R.E. et al., Science, 242, 423-426 (1988); Boss, M.A. et al., Nucl. Acids Res., 12, 3791-3806 (1984); Bukovsky, J. et al., Hybridoma, 6, 219-228 (1987); Daino, M. et al., Anal. Biochem., 166, 223-229 (1987); Huston, J.S. et al., Proc. Natl. Acad. Sci. USA, 85, 5879-5883 (1988); Jones, P.T. et al., Nature, 321, 522-525 (1986); Langone, J.J. et al. (ed.), "Methods in Enzymology", Vol. 121 (Immunochemical Techniques, Part I: Hybridoma Technology and Monoclonal Antibodies), Academic Press, New York (1986); Morrison, S. et al., Proc. Natl. Acad. Sci. USA, 81, 6851-6855 (1984); Oi, V.T. et al., BioTechniques, 4, 214-221 (1986); Riechmann, L. et al., Nature, 332, 323-327 (1988); Tramontano, A. et al., Proc. Natl. Acad.Sci. USA, 83, 6736-6740 (1986); Wood, C. et al., Nature, 314, 446-449 (1985); Nature, 314, 452-454 (1985) and references cited in the foregoing references (descriptions in the above references are incorporated in the specification by reference).

As the anti-MT-MMP monoclonal antibody used in the present invention, any monoclonal antibody that can specifically recognize MT-MMP may be used. As MT-MMP as the antigen used for the production of the anti-MT-MMP monoclonal antibody, 6 types of MT1-MMP, MT2-MMP, MT3-MMP, MT4-MMP, MT5-MMP, and MT6-MMP are known so far, and it is necessary that the anti-MT-MMP monoclonal antibody can specifically recognize at least one kind, preferably only one kind of the antigens. An antigen belonging to the class of MT-MMP may exist besides the aforementioned six types of MT-MMPs, and a monoclonal antibody that can recognize such antigen can also be used.

A monoclonal antibody obtained by applying the cell fusion technique using a myeloma cell (Kohler, G. and Milstein, C., Nature, 256, 495-497 (1975) and the like) can be used as the anti-MT-MMP monoclonal antibody. For example, one or more kinds of antibodies can be used which are selected from the group consisting of anti-MT1-MMP monoclonal antibody, an anti-MT2-MMP monoclonal antibody, an anti-MT3-MMP monoclonal antibody, an anti-MT4-MMP monoclonal antibody, an anti-MT5-MMP monoclonal antibody and an anti-MT6-MMP monoclonal antibody, which are produced by a known method using at least one kind of MT-MMPs, preferably a specific MT-MMP or a fragment containing an antigenic determinant thereof as an antigen. An anti-MT1-MMP monoclonal antibody is more preferred. Some of these antibodies are commercially available and can be easily obtained. Further, as the anti-MT-MMP monoclonal antibody to be bound with the lipid membrane structure, a $F(ab')_2$ fragment, Fab' fragment or Fab fragment of an anti-MT-MMP monoclonal antibody can be preferably used, and a Fab' fragment can be more preferably used. Further, a humanized Fab' fragment is also preferred. A ratio of the anti-MT-MMP monoclonal antibody to be added on the basis of a total lipid amount of the lipid membrane structure is preferably 1:0.00001 to 1:0.25, more preferably 1:0.0001 to 1:0.2, further preferably 1:0.0001 to 1:0.01 in terms of molar ratio. When the lipid derivative having a maleinimide structure is contained in the lipid membrane structure, the ratio thereof in terms of molar ratio on the basis of the maleinimide group (antibody: maleinimide group) is preferably 1:0.01 to 1:20, more preferably 1:0.25 to 1:4.5, further preferably 1:1 to 1:3. The above ranges are mentioned only as examples, and the amounts should not be necessarily limited to these ranges.

In order that the lipid membrane structure of the present invention containing the anti-MT-MMP monoclonal antibody exhibits the aforementioned superior effects, it is desirable that the lipid membrane structure does not aggregate and has blood retention. For prevention of the aggregation, the amount of the anti-MT-MMP monoclonal antibody to be added and/or the content of the lipid derivative for allowing the anti-MT-MMP monoclonal antibody to be present on or to bind to the surface of the membrane of the lipid membrane structure (e.g., lipid derivative having a maleinimide structure) can be suitably determined. When a lipid derivative having a maleinimide structure is contained in the lipid membrane structure, the amount of the lipid derivative to be added may be those mentioned above.

When the pharmaceutical composition of the present invention comprising the lipid membrane structure containing an anti-MT-MMP monoclonal antibody and a medicinally active ingredient and/or a gene is used, the anti-MT-MMP monoclonal antibody contained in the lipid membrane structure containing the anti-MT-MMP monoclonal antibody specifically and selectively reacts with MT-MMP. It is known that MT-MMP is actively expressed in certain types of tumor cells and also involved in angiogenesis. However, whether or not MT-MMP is expressed in a neoplastic vessel has not been fully clarified. When the pharmaceutical composition of the present invention is administered to an animal such as a human or experimental cells, a medicinally active ingredient and/or a gene can be efficiently delivered to the tumor cells. Examples of tumor cells expressing MT-MMP include, for example, cells of fibrosarcoma, squamous carcinoma, neuroblastoma, breast carcinoma, gastric cancer, hepatoma, bladder cancer, thyroid tumor, urinary tract epithelial cancer, glioblastoma, acute myeloid leukemia, pancreatic duct cancer, prostate cancer and the like, but not limited to these cells. When the pharmaceutical composition is administered to an animal such as a human or experimental cells, a medicinally active ingredient and/or a gene can be efficiently delivered to an angiogenesis front inside a tumor. Examples of the angiogenesis front inside a tumor include endothelial cells of ruffling edge and the like, but not limited to these examples.

The pharmaceutical composition of the present invention comprises the lipid membrane structure containing an anti-MT-MMP monoclonal antibody and a medicinally active ingredient and/or a gene, and the form thereof is not particularly limited. For example, besides a form of a simple mixture of the aforementioned lipid membrane structure and the medicinally active ingredient and/or gene, the composition may have a form in which the medicinally active ingredient and/or gene is retained by the aforementioned lipid membrane structure. The term "retain" used herein means that the medicinally active ingredient and/or gene are present in a lipid membrane, on a surface of lipid membrane, in a internal space of lipid membrane, in a lipid layer and/or on a surface of lipid layer of the lipid membrane structure. Considering that the composition is administered to an animal such as a human, the pharmaceutical composition of the present invention is preferably in the form in which the medicinally active ingredient and/or gene is retained by the aforementioned lipid membrane structure. In the pharmaceutical composition of the present invention, the amount of the medicinally active ingredient and/or gene is not particularly limited, and the amount may be that sufficient for effectively expressing pharmacological activity thereof in an organism (or in cells). The type of the medicinally active ingredient and/or gene is not also particularly limited, and may be suitably determined depending on a type of disease to be treated and/or prevented, a purpose of therapeutic or prophylactic treatment, a form of the lipid membrane structure, and the like.

Although the type of the medicinally active ingredient contained in the pharmaceutical composition of the present invention should not be particularly limited, examples include an antitumor agent, an immunostimulator, a cytokine having an antitumor effect, a contrast medium, or the like. Examples of the antitumor agent include, for example, camptothecin derivatives such as irinotecan hydrochloride, nogitecan hydrochloride, exatecan, RFS-2000, lurtotecan, BNP-1350, Bay-383441, PNU-166148, IDEC-132, BN-80915, DB-38, DB-81, DB-90, DB-91, CKD-620, T-0128, ST-1480, ST-1481, DRF-1042 and DE-310, taxane derivatives such as docetaxel hydrate, paclitaxel, IND-5109, BMS-184476, BMS-188797, T-3782, TAX-1011, SB-RA-31012, SBT-1514 and DJ-927, ifosfamide, nimustine hydrochloride, carboquone, cyclophosphamide, dacarbazine, thiotepa, busulfan, melphalan, ranimustine, estramustine phosphate sodium, 6-mercaptopurine riboside, enocitabine, gemcitabine hydrochloride, carmofur, cytarabine, cytarabine ocphosphate, tegafur, doxifluridine, hydroxycarbamide, fluorouracil, methotrexate, mercaptopurine, fludarabine phosphate, actinomycin D, aclarubicin hydrochloride, idarubicin hydrochloride, epirubicin hydrochloride, daunorubicin hydrochloride, doxorubicin hydrochloride, pirarubicin hydrochloride, bleomycin hydrochloride, zinostatin stimalamer, neocarzinostatin, mytomycin C, bleomycin sulfate, peplomycin sulfate, etoposide, vinorelbine tartrate, vincristine sulfate, vindesine sulfate, vinblastine sulfate, amrubicin hydrochloride, gefitinib, exemestan, capecitabine, TNP-470, TAK-165, KW-2401, KW-2170, KW-2871, KT-5555, KT-8391, TZT-1027, S-3304, CS-682, YM-511, YM-598, TAT-59, TAS-101, TAS-102, TA-106, FK-228, FK-317, E7070, E7389, KRN-700, KRN-5500, J-107088, HMN-214, SM-11355, ZD-0473 and the like, and the examples of the contrast medium include, for example, sodium amidotrizoate/meglumine, meglumine amidotrizoate, ioxaglic acid, ioxilan, iodixanol, sodium iolactamate, meglumine iotroxate, iotrolan, iopanoic acid, iopamidol, iopromido, iohexol, ioversol, iomeprol, and the like.

The gene contained in the pharmaceutical composition of the present invention may be any of oligonucleotide, DNA, and RNA, and in particular, examples thereof include a gene for in vitro gene introduction such as transformation and a gene that act upon in vivo expression, for example, a gene for gene therapy, and the like. Examples of the gene for gene therapy include an antisense oligonucleotide, antisense DNA, antisense RNA, gene coding for a physiologically active substance such as enzymes and cytokines, and the like, and among them, a gene is preferred of which gene product has an antitumor effect.

When the pharmaceutical composition of the present invention contains a gene, it is preferable to add a compound having a gene transfer function as a component of the lipid membrane structure containing an anti-MT-MMP monoclonal antibody to efficiently introduce the gene into a cell. Examples of such compounds include O,O'-N-didodecanoyl-N-($\alpha$-trimethylammonioacetyl)-diethanolamine chloride, O,O'-N-ditetradecanoyl-N-($\alpha$-trimethylammonioacetyl)-diethanolamine chloride, O,O'-N-dihexadecanoyl-N-($\alpha$-trimethylammonioacetyl)-diethanolamine chloride, O,O'-N-dioctadecenoyl-N-($\alpha$-trimethylammonioacetyl)-diethanolamine chloride, O,O',O"-tridecanoyl-N-($\omega$-trimethylammoniodecanoyl)aminomethane bromide, N-[$\alpha$-trimethylammonioacetyl]-didodecyl-D-glutamate, dimethyldioctadecylammonium bromide, 2,3-dioleoyloxy-N-[2-(sperminecarboxamido)ethyl]-N,N-dimethyl-1-propaneammonium trifluoroacetate, 1,2-dimyristyloxypropyl-3-dimethylhydroxyethylammonium bromide, 3-β-[N-(N',N'-dimethylaminoethane)carbamoyl]cholesterol, and the like. A form is preferred in which any of the compounds having the gene transfer function is present (binds) in a membrane, on a surface of membrane, in a internal space of membrane, in a lipid layer and/or on a surface of lipid layer of the lipid membrane structure.

The pharmaceutical composition of the present invention can be prepared by adding a medicinally active ingredient and/or a gene to the lipid membrane structure, and the composition can be used as a pharmaceutical composition for therapeutic treatment and/or prevention of any of various diseases involving MT-MMP, preferably tumor or cancer. When a gene is contained, the composition can also be used as a gene delivery kit. The existing form of the pharmaceutical composition of the present invention and methods for preparation thereof are not particularly limited, and the composition may be produced in the same form as the aforementioned lipid membrane structure. For example, examples of the form include a dried mixture form, a form of dispersion in an aqueous solvent, and a form obtained by drying or freezing the previously mentioned form.

The form of dried mixture can be produced by first dissolving the components of the lipid membrane structure containing an anti-MT-MMP monoclonal antibody and a medicinally active ingredient and/or a gene in an organic solvent such as chloroform and then subjecting the resulting mixture to solidification under reduced pressure by using an evaporator or spray drying by using a spray dryer. Examples of the form of dispersion in an aqueous solvent include, but not limited to, multi-lamella liposomes, unilamella liposomes, O/W type emulsions, W/O/W type emulsions, spherical micelles, fibrous micelles, layered structures of irregular shapes and the like. The size of particles (particle diameter) as the mixture, a composition of the aqueous solvent and the like are not particularly limited. For example, liposomes may have a size of 50 nm to 5 μm, preferably 50 to 400 nm, more preferably 50 to 200 nm, still more preferably 50 nm to 150 nm, spherical micelles may have a size of 5 to 100 nm, and emulsions may have a particle diameter of 50 nm to 5 μm. The particle diameter means a weight average particle diameter determined by the quasi-elastic light scattering method. The concentration of the mixture in the aqueous solvent is also not particularly limited. Several methods are known as methods for producing a mixture of lipid membrane structures and a medicinally active ingredient and/or a gene in the form of dispersion in an aqueous solvent. It is possible to appropriately chose a suitable method depending on the existing form of the mixture of the lipid membrane structure containing an anti-MT-MMP monoclonal antibody and a medicinally active ingredient and/or a gene as follows.

Production Method 1

Production Method 1 is a method of adding an aqueous solvent to the aforementioned dried mixture and emulsifying the mixture by using an emulsifier such as homogenizer, ultrasonic emulsifier, high-pressure injection emulsifier, or the like. When it is desired to control the size (particle diameter), extrusion can be further performed under a high pressure by using a membrane filter having uniform pore sizes. In this method, in order to prepare a dried mixture of components of the lipid membrane structure containing an anti-MT-MMP monoclonal antibody and a medicinally active ingredient and/or a gene first, it is necessary to dissolve the lipid membrane structure containing an anti-MT-MMP monoclonal antibody and a medicinally active ingredient and/or a gene in an organic solvent, and the method has an advantage that it can make the best utilization of interactions between the a medicinally active ingredient and/or a gene and components of the lipid membrane structure. More specifically, even when the lipid membrane structures have a layered structure, a medicinally active ingredient and/or a gene can enter into the inside of the multiple layers, and thus use of this method generally provides a higher retention ratio of the medicinally active ingredient and/or a gene in the lipid membrane structures.

Production Method 2

Production Method 2 is a method of adding an aqueous solvent containing a medicinally active ingredient and/or a gene to dried components of the lipid membrane structure containing an anti-MT-MMP monoclonal antibody obtained by dissolving the components in an organic solvent and evaporating the organic solvent, and emulsifying the mixture to attain the production. When it is desired to control the size (particle diameter), extrusion can be further performed under a high pressure by using a membrane filter having uniform pore sizes. This method can be used for a medicinally active ingredient and/or a gene that is hardly dissolved in an organic solvent, but can be dissolved in an aqueous solvent. When the lipid membrane structures are liposomes, they have an advantage that they can retain a medicinally active ingredient and/or a gene also in the part of internal aqueous phase.

Production Method 3

Production Method 3 is a method of further adding an aqueous solvent containing a medicinally active ingredient and/or a gene to lipid membrane structures containing an anti-MT-MMP monoclonal antibody such as liposomes, emulsions, micelles or layered structures already dispersed in an aqueous solvent. This method is limitedly applied to a water-soluble medicinally active ingredient and/or gene. In this method, the addition of a medicinally active ingredient and/or a gene to already prepared lipid membrane structures is performed from the outside. Therefore, when the medicinally active ingredient and/or gene is a polymer, the medicinally active ingredient and/or gene may not enter into the inside of the lipid membrane structures, and the medicinally active ingredient and/or a gene may be present in a form that it binds to the surfaces of lipid membrane structures. It is known that when liposomes are used as the lipid membrane structures, use of Production Method 3 may result in formation of a sandwich-like structure in which the medicinally active ingredient and/or gene is sandwiched between liposome particles (generally called as a complex). An aqueous dispersion of lipid membrane structures alone is prepared beforehand in this production method. Therefore, decomposition of a medicinally active ingredient and/or a gene during the emulsification need not be taken into consideration, and a control of the size (particle diameter) is also readily operated, which enables relatively easier preparation compared with Production Methods 1 and 2.

Production Method 4

Production Method 4 is a method of further adding an aqueous solvent containing a medicinally active ingredient and/or a gene to a dried product obtained by once producing lipid membrane structures containing an anti-MT-MMP monoclonal antibody dispersed in an aqueous solvent and then drying the same. In this method, the medicinally active ingredient and/or gene is limited to a water-soluble medicinally active ingredient and/or a gene as in Production Method 3. A significant difference from Production Method 3 is a mode of presence of the lipid membrane structures and the medicinally active ingredient and/or gene. That is, in Production Method 4, lipid membrane structures dispersed in an aqueous solvent are once produced and further dried to obtain a dried product, and at this stage, the lipid membrane structures are present in a state of a solid as fragments of lipid membranes. In order to allow the fragments of lipid membranes to be present in a solid state, it is preferable to use a solvent added with a sugar (aqueous solution), preferably sucrose (aqueous solution) or lactose (aqueous solution), as the aqueous solvent as described above. In this method, when the aqueous solvent containing a medicinally active ingredient and/or a gene is added, hydration of the fragments of the lipid membranes present in a state of a solid quickly starts with the invasion of water, and thus the lipid membrane structures can be reconstructed. At this time, a structure of a form in which a medicinally active ingredient and/or a gene is retained in the inside of the lipid membrane structures can be produced.

In Production Method 3, when the medicinally active ingredient and/or gene is a polymer, the medicinally active ingredient and/or gene cannot enter into the inside of the lipid membrane structures, and is present in a mode that it binds to the surfaces of the lipid membrane structures. Production Method 4 significantly differs in this point. In Production Method 4, an aqueous dispersion of lipid membrane structures alone is prepared beforehand, and therefore, decomposition of the medicinally active ingredient and/or gene during the emulsification need not be taken into consideration, and a control of the size (particle diameter) is also easily attainable. For this reason, said method enables relatively easier preparation compared with Production Methods 1 and 2. Besides the above mentioned advantages, this method also has advantages that storage stability for a pharmaceutical preparation (or pharmaceutical composition) is easily secure, because the method uses lyophilization or spray drying; when the dried preparation is rehydrated with an aqueous solution of a medicinally active ingredient and/or a gene, original size (particle diameter) can be reproduced; even when a polymer medicinally active ingredient and/or gene is used, the medicinally active ingredient and/or gene can be easily retained in the inside of the lipid membrane structures and the like.

As other method for producing a mixture of lipid membrane structures and a medicinally active ingredient and/or a gene in a form of a dispersion in an aqueous solvent, a method well known as that for producing liposomes, e.g., the reverse phase evaporation method or the like, may be separately used. When it is desired to control the size (particle diameter), extrusion can be performed under a high pressure by using a membrane filter having uniform pore sizes. Further, examples of the method for further drying a dispersion, in which the aforementioned mixture of lipid membrane structures and a medicinally active ingredient and/or a gene is dispersed in an aqueous solvent, include lyophilization and spray drying. As the aqueous solvent in this process, it is preferable to use the aforementioned solvent added with a sugar (as an aqueous solution), preferably sucrose (as an aqueous solution) or lactose (as an aqueous solution). Examples of the method for further freezing a dispersion, in which the aforementioned mixture of lipid membrane structures and a medicinally active ingredient and/or a gene is dispersed in an aqueous solvent, include ordinary freezing methods. As the aqueous solvent in this process, it is preferable to use a solvent added with a sugar (as an aqueous solution) or polyhydric alcohol (aqueous solution).

Production Method 5

By producing lipid membrane structures using components of the lipid membrane structures other than the anti-MT-MMP monoclonal antibody (including a lipid derivative that can react with mercapto group in the anti-MT-MMP monoclonal antibody (preferably, Fab fragment, F(ab')$_2$ fragment, Fab' fragment of the antibody or the like) and a medicinally active ingredient and/or a gene and then adding the anti-MT-MMP monoclonal antibody in a manner similar to any of those of Production Methods 1 to 4, a composition in a form where the anti-MT-MMP monoclonal antibody is present on (or binds to) the surfaces of the membranes of lipid membrane structures can be produced.

Production Method 6

By producing lipid membrane structures using components of the lipid membrane structures other than the anti-MT-MMP monoclonal antibody and a lipid derivative that can react with mercapto group in the anti-MT-MMP monoclonal antibody (preferably, Fab fragment, F(ab')$_2$ fragment, Fab' fragment of the antibody or the like) and a medicinally active ingredient and/or a gene and then adding the anti-MT-MMP monoclonal antibody and the lipid derivative that can react with mercapto group in the anti-MT-MMP monoclonal antibody in a manner similar to any of those of Production Methods 1 to 4, a composition in a form where the anti-MT-MMP monoclonal antibody is present on (or binds to) the surfaces of the membranes of lipid membrane structures can be produced.

Lipids which can be added to the pharmaceutical composition of the present invention may be suitably chosen depending on a type of a medicinally active ingredient and/or a gene and the like to be used. When a medicinally active ingredient is used, the lipids are used in an amount of, for example, 0.1 to 1000 parts by mass, preferably 0.5 to 200 parts by mass, in terms of the total lipid amount, on the basis of 1 part by mass of the medicinally active ingredient. When a gene is used, the amount is preferably 1 to 500 nmol, more preferably 10 to 200 nmol, in terms of the total lipid amount, on the basis of 1 $\mu$ g of the gene.

The administration method of the pharmaceutical composition containing the lipid membrane structures of the present invention is not particularly limited, and either oral administration or parenteral administration may be used. Examples of dosage forms for oral administration include, for example, tablets, powders, granules, syrups, capsules, solutions for internal use and the like, and examples of dosage forms for parenteral administration include, for example, injections, drip infusion, eye drops, ointments, suppositories, suspensions, cataplasms, lotions, aerosols, plasters and the like. Injection or drip infusion is preferred among them, and administration methods include intravenous injection, arterial injection, subcutaneous injection, intradermal injection and the like, as well as local injection to targeted cells or organs.

Examples

The present invention will be explained more specifically with reference to the following examples. However, the scope of the present invention is not limited to these examples.

Example 1 : Measurement of anti-MT1-MMP monoclonal antibody-binding liposomes 1. Preparation of liposomes not containing antibody

Liposomes of the 4 kinds of formulations shown in Table 1 were prepared. To all the formulations, a fluorescent lipid, (2-(6-(7-nitrobenz-2-oxa-1,3-diazol-4-yl)amino)hexanoyl-1-hexadecanoyl-sn-glycero-3-phosphocholine, NBD-C$_6$-HPC), was added as a liposome marker.

The formulation without encapsulation of an anticancer agent (doxorubicin, DOX) for observation of adsorption (in vitro) and transfer (in vivo) of empty liposomes to cancer cells (Formulations 1 and 2) and the formulation with encapsulation of the anticancer agent for pharmacological experiments (Formulations 3 and 4) were separately prepared, and negative control samples (Formulations 1 and 3) not containing antibody-binding lipid (poly(ethylene glycol)- $\alpha$ -distearoylphosphatidylethanolamine-m-maleinimide, DSPE-PEG-MAL) were prepared as reference groups.

Hydrogenated soybean phosphatidylcholine (HSPC) and cholesterol (Chol) were weighed and dissolved in an appropriate volume of a mixture of chloroform and methanol (3:1) and added with NBD-C$_6$-HPC dissolved in methanol at a concentration of 5 mg/mL. The organic solvents were evaporated by using an evaporator, and the residue was further dried under reduced pressure for 1 hour. Then, the dried lipids (lipid film) were added with 155 mM aqueous ammonium sulfate (pH 5.5) heated at 65°C beforehand, and the mixture was lightly stirred by using a vortex mixer (until lipids were substantially peeled off from a recovery flask). The mixture was prepared so that the concentrations of the lipids including the fluorescent lipid at this time point became as follows: HSPC: 28.2 mM, Chol: 19.2 mM, and NBD-C$_6$-HPC: 0.2 mg/mL. Then, this lipid dispersion was transferred to a homogenizer, homogenized for 10 strokes and sized by using polycarbonate membrane filters with various pore sizes (0.2 $\mu$ m x 2 times, 0.1 $\mu$ m x 2 times and 0.05 $\mu$ m x 2 times) to prepare a dispersion of empty liposomes having a particle diameter of about 100 nm.

This empty liposome dispersion was diluted 5 times with physiological saline, and the resulting diluted liposome dispersion was placed in an ultracentrifugation tube and centrifuged at 65,000 rpm for 1 hour. Then, the supernatant was discarded, and the precipitates were resuspended in physiological saline to make the dispersion volume the volume of the liposome dispersion before the dilution. The empty liposome dispersion in which the external aqueous phase was replaced with physiological saline as described above was divided into 2 groups for use as empty liposomes and for encapsulating a medicament.

The method for encapsulating a medicament will be explained. The empty liposome dispersion and a DOX solution (medicament concentration: 3.3 mg/mL physiological saline) were heated beforehand at 65°C, and the empty liposome dispersion and the DOX solution were added at a volume ratio of 4:6 (i.e., final medicament concentration: 2.0 mg/mL) and incubated at 65°C for 1 hour.

Each of the empty liposome group and the medicament encapsulating liposome group was divided into two groups. To one group, only N-{carbonyl-methoxypolyethylene glycol-2000}-1,2-distearoyl-sn-glycero-3-phosphoethanolamine (DSPE-PEG) was added so that the membrane compositions shown in Table 1 was obtained (Formulations 1 and 3), and to the other group, DSPE-PEG and DSPE-PEG-MAL were added so that the membrane compositions shown in Table 1 was obtained (Formulations 2 and 4). These substances were added as powders, and the mixtures were incubated at 65°C for 10 minutes.

2. Measurement of physical properties of liposomes

(1) Encapsulation efficiency of doxorubicin into liposomes

A part of each of the aforementioned liposome dispersions (Formulations 3 and 4) was sampled and subjected to gel filtration (Sephadex G-50, mobile phase: physiological saline), and the encapsulating efficiency was obtained by quantifying doxorubicin in the liposome fraction eluted in the void volume by using a fluorescence detector. The rate of encapsulation of the medicament of each formulation was substantially 100%.

(2) Particle size

A part of each of the aforementioned liposome dispersions (Formulations 1 to 4) was sampled, and particle size was measured by the quasi-elastic light scattering (QELS) method. As a result, the size was about 100 nm in all of the dispersions. The particle size was also measured for the liposomes added with the antibodies, and the particle size was found to be about 100 nm in all of the dispersions.

Table 1

| Formu-lation | DOX encapsulation | | Lipids other than fluorescent lipids | | |
|---|---|---|---|---|---|
| | Present / Absent | Conc. (mg/mL) | Present/Absent DSPE-PEG-MAL | Composition of lipids (mM) | Total Conc.(mM) |
| 1 | Absent | - | Absent | HSPC/Chol/DSPE-PEG (28.2/19.2/1.3) | 48.7 |
| 2 | | | Present | HSPC/Chol/DSPE-PEG/DSPE-PEG-MAL[a)] (28.2/19.2/1.04/0.26) | 48.7 |
| 3 | Present | 2 | Absent | HSPC/Chol/DSPE-PEG (11.28/7.68/0.52) | 19.48 |
| 4 | | | Present | HSPC/Chol/DSPE-PEG/DSPE-PEG-MAL[a)] (11.28/7.68/0.416/0.104) | 19.48 |

a) Cat. No. 172D0F02 (SHEARWATER) b) Cat. No. N-3786 (Molecular Probes, Inc.)

b) NBD-$C_6$-HPC Cat. No. N-3786 (Molecular Probes, Inc.)

The meaning of the abbreviations used in Example 2 and the following examples are shown below.
Fab'-DOX-LP: Liposomes bound with anti-MT1-MMP monoclonal antibody and encapsulating anticancer agent
Fab'-LP: Liposomes bound with anti-MT1-MMP monoclonal antibody
DOX-LP: Liposomes encapsulating anticancer agent (liposome not introduced with maleinimide group)
LP: Liposomes (liposomes not introduced with maleinimide group)
DOX-LP-mal: Liposomes introduced with maleinimide group and encapsulating anticancer agent
LP-mal: Liposomes introduced with maleinimide group

Example 2: Preparation of anti-MT1-MMP monoclonal antibody-binding liposomes

1) Production and purification of IgG

Anti-MT1-MMP monoclonal antibody-producing hybridoma cells obtained according to the method described in WO02/041000A1 were cultured in RPMI 1640 medium containing 5% fetal bovine serum to obtain $1.0 \times 10^8$ cells. The cells were suspended in the medium at a density of $1.0 \times 10^7/0.5$ mL and intraperitoneally administered to mice (Balb/c type, female, 6-week old) which were intraperitoneally administered beforehand with pristane one week before the date. Ascites was extracted from ten mice on the 7th and 9th day to obtain 18 mL of ascites.

The resulting ascites was centrifuged to remove insoluble solids and precipitates, and gradually added with solid ammonium sulfate to a concentration of 40% saturation. After the addition, stirring was continued for 2 hours. The precipitates were collected by centrifugation and dissolved with a small amount of 1.5 M glycine-NaOH buffer (pH 8.9) containing 0.5 M NaCl. This solution was placed in a dialysis tube and dialyzed against 1.5 M glycine-NaOH buffer (pH 8.9) containing 0.5 M NaCl. After the dialysis, the precipitate was removed by centrifugation, and the volume and A280 of the supernatant were measured to estimate that the amount of the protein obtained was 140 mg/12.5 mL.

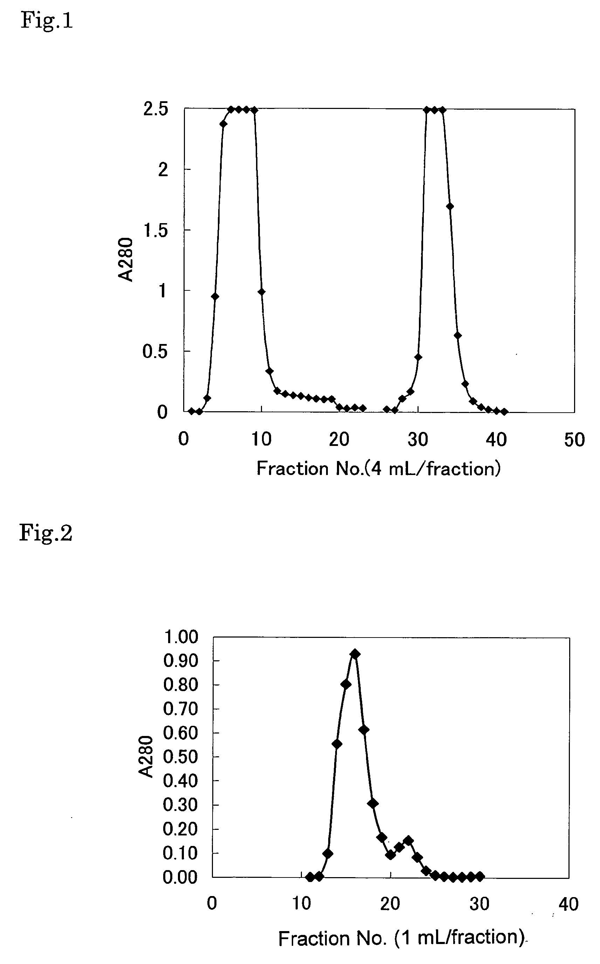

The centrifuged supernatant was loaded on a recombinant protein A Sepharose FF gel column (diameter: 2.5 cm x length: 5.9 cm) equilibrated with 1.5 M glycine-NaOH buffer (pH 8.9) containing 0.5 M NaCl and washed with 1.5 M glycine-NaOH buffer (pH 8.9) containing 0.5 M NaCl. The centrifuged supernatant and washing solution passed through the column were collected as 4-mL fractions, and A280 was measured for the fractions of the fraction Nos. 1 to 23. The A280 was confirmed to become 0.05 or less, and then the adsorbed protein was eluted with 0.1 M citrate buffer (pH 5.0). The eluate was collected as fractions in a volume of 4 mL each in test tubes to which 0.5 mL of 3 M Tris-HCl buffer (pH 7.5) was added beforehand, and A280 was continually measured for fraction Nos. 26 to 41. Fig. 1 shows the results of the affinity purification of IgG. Fraction Nos. 29 to 36 were collected and pooled as IgG. The resulting IgG fractions were placed in a dialysis tube and dialyzed against 0.1 M phosphate buffer (pH 7.0). The dialyzed fractions were concentrated by using Ultra Filter UK-50. The IgG fractions were estimated to be 62 mg/6 mL on the basis of the A280 measurement of the concentrated fractions. The IgG concentration was adjusted to 10 mg/mL and cryopreserved as 1-mL aliquots.

2) Fab'-fragmentation of IgG

A volume of 1 mL of the purified IgG purified and adjusted to 10 mg/mL in 1) mentioned above was sampled and dialyzed against 0.1 M sodium acetate buffer (pH 4.2) containing 0.1 M NaCl, and then added with pepsin at a concentration of 2% (w/w) based on the amount of antibodies and digested at 37°C for 20 hours. The digested product was added with 0.2 mL of 3 M Tris-HCl buffer (pH 7.5) to terminate the reaction. The whole digestion product was loaded on a Ultrogel AcA44 gel filtration column (diameter: 1.5 cm x length: 47 cm) equilibrated with 0.1 M phosphate buffer (pH 7.0) and collected as 1-mL fractions, and A280 was measured for the fraction Nos. 11 to 30. Fig. 2 shows the results of the gel filtration of $F(ab')_2$ fraction. The fraction Nos. 13 to 18 were collected and pooled as an $F(ab')_2$ fraction. The resulting $F(ab')_2$ fraction was concentrated to 0.46 mL by using Centricon-30. A280 of the concentrated fraction was measured, and the amount of the resulting $F(ab')_2$ was estimated to be 3.4 mg.

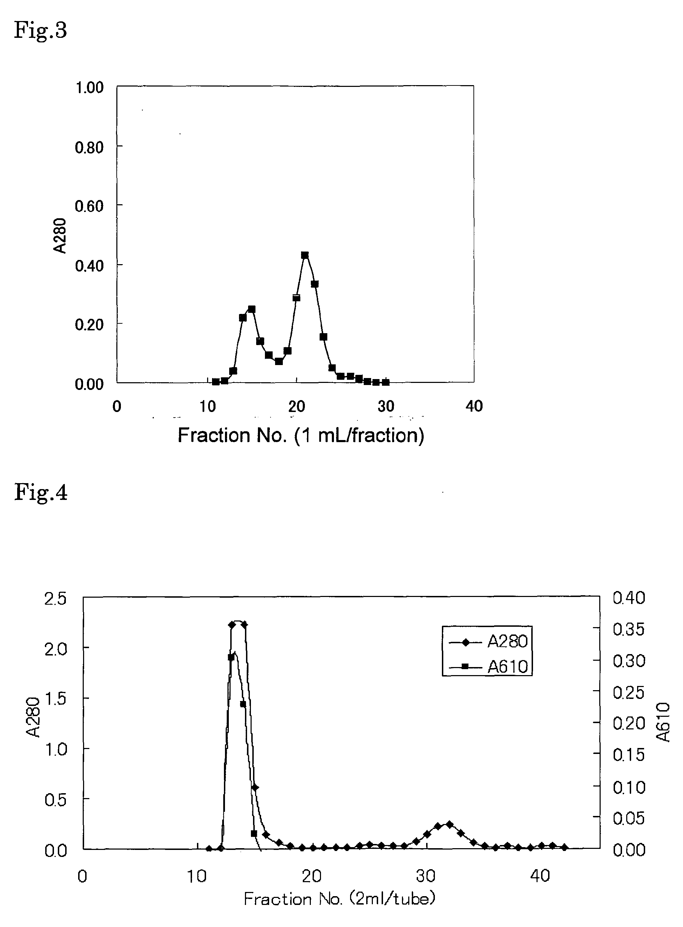

The resulting $F(ab')_2$ was adjusted to a volume of 0.9 mL with 0.1 M phosphate buffer (pH 6.0), added with 0.1 mL of 0.1 M cysteamine hydrochloride (final concentration: 0.01 M) and thereby reduced at 37°C for 1.5 hours. The resultant was loaded on a Ultrogel AcA44 gel filtration column (diameter: 1.5 cm x length: 47 cm) equilibrated with PBS containing 5 mM EDTA and collected as 1-mL fractions, and A280 was measured for the fractions of the fraction Nos. 11 to 30. Fig. 3 shows the results of the gel filtration of the Fab' fraction. The fractions of the fraction Nos. 19 to 23 were collected and pooled a Fab' fraction. The resulting Fab' fraction was concentrated to 0.56 mL by using Centricon-30. A280 of the concentrated fraction was measured, and the amount of the resulting Fab' was estimated to be 1.5 mg.

3) Preparation of anti-MT1-MMP monoclonal antibody-binding liposomes

(Preparation Examples ① to ⑦)

To the Fab' fraction (1.96 mg/0.37 mL) prepared in 2) mentioned above, 0.41 mL of the maleinimide group-introduced and anticancer agent (doxorubicin (DOX))-encapsulating liposomes (DOX-LP-mal, maleinimide concentration: 104 nmol/mL) of the Formulation 4 mentioned in Table 1 was added so that the maleinimide molar ratio became 1:1 and mixed. The reaction was continued for 20 hours in a low temperature chamber under light shielding, and then unreacted mercapto groups were blocked with N-ethylmaleinimide in an amount of 10 times of the amount of Fab' in terms of molar amount (4.26 μL of 0.1 M aqueous solution was added). The reaction mixture was loaded on a Sepharose CL-4B column (diameter: 1.5 cm x length: 47 cm) equilibrated with PBS and collected as 2-mL fractions, and A280 (reflecting the protein concentration) was measured for the fraction Nos. 11 to 42, and A610 (reflecting turbidity, i.e., the lipid concentration) was measured for the fraction Nos. 11 to 20. Fig. 4 shows the results of elution in the gel filtration of the above procedure. The fraction Nos. 13 and 14 were collected and pooled as an anti-MT1-MMP antibody-binding and anticancer agent-encapsulating liposomes (Fab'-DOX-LP) fraction to obtain Fab'-DOX-LP (Preparation Example (1), dilution ratio was 10% as measured by using DOX as index). Unreacted Fab' was eluted in the fraction Nos. 29 to 35, and thus it was confirmed that the liposome fraction and the unreacted Fab' fraction were separated by the gel filtration.

In a similar manner, each Fab'-DOX-LP (Preparation Example ②, dilution ratio was 9.2% as measured by using DOX as index), Preparation Example ③ (dilution ratio was 12% as measured by using DOX as index), and Preparation Example ④ (dilution ratio was 3.9% as measured by using DOX as index) was prepared. Further, using the maleinimide group-introduced liposomes (LP-mal, maleinimide concentration: 260 nmol/mL) of Formulation 2 mentioned in Table 1, each anti-MT1-MMP antibody-binding liposomes (Fab'-LP) not encapsulating anticancer agent (Preparation Example ⑤, dilution ratio was 3.7% as measured by using HSPC as index), Preparation Example ⑥ (dilution ratio was 4.4% as measured by using HSPC as index) and Preparation Example ⑦ (dilution ratio was 2.1% as measured by using HSPC as index) was prepared in a manner similar to that mentioned above.

The dilution ratios relative to the liposomes as the starting material mentioned in the preparation examples were calculated by multiplying the cumulative A610 value of the fraction pooled as the antibody-binding liposomes (calculated from the A610 value which was determined at the time of the gel filtration after the binding of antibody)/the cumulative A610 value of void fraction, with each charged volume of the starting material/the volume of liposomes in each preparation example. The phospholipid concentration of the anti-MT1-MMP antibody-binding liposomes was calculated by multiplying the phospholipid concentration of LP-mal of Formulation 2 mentioned in Table 1 or DOX-LP-mal of Formulation 4 mentioned in Table 1 (measured by using Phospholipid B-Test Wako (Wako Pure Chemical Industries), and a value corresponding to the influence of DOX per se on the measurement system was subtracted) with the aforementioned dilution ratio.

Further, as for the liposomes used as liposomes not bound with antibody in the test examples mentioned below (liposomes not introduced with maleinimide group, LP), or the anticancer agent-encapsulating liposomes (liposomes not introduced with maleinimide group, DOX-LP), the phospholipid concentrations of the liposomes of Formulation 1 or Formulation 3 not introduced with maleinimide group mentioned in Table 1 were measured, and the liposomes were diluted with PBS before use so that the phospholipid concentration became the same as that of the corresponding anti-MT1-MMP monoclonal antibody-binding liposomes.

Example 3: Preparation of anti-MT1-MMP monoclonal antibody-binding liposomes

(Preparation Example ⑧)

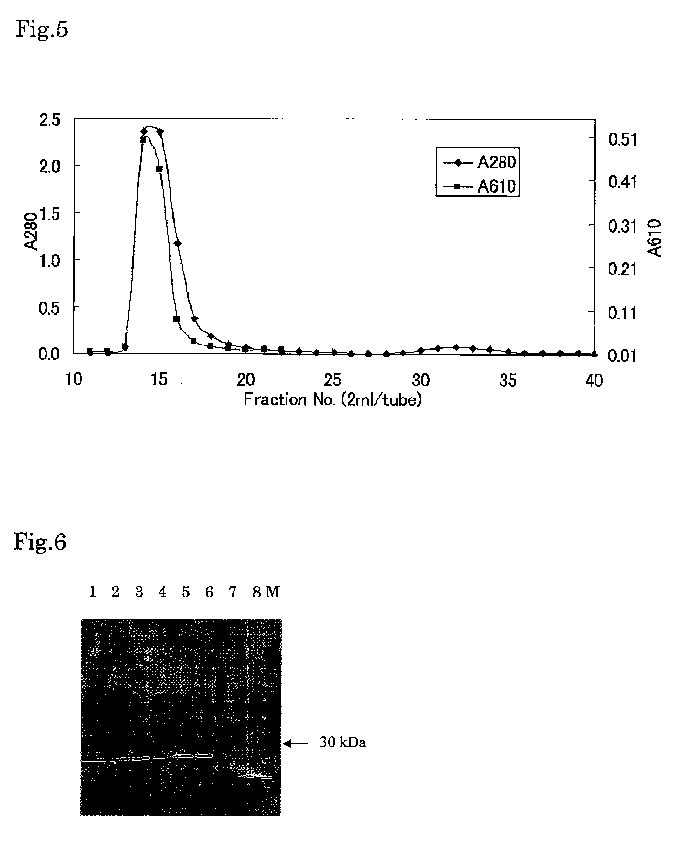

In the same manner as that in Example 2, Fab'-DOX-LP (Preparation Example ⑧, dilution ratio was 14% as measured by using DOX as index) was obtained from DOX-LP-mal of Formulation 4 mentioned in Table 1 (maleinimide concentration: 100 nmol/mL), except that the maleinimide molar ratio of the Fab' fraction and the maleinimide group-introduced liposomes was adjusted to 1:3. Fig. 5 shows the results of elution in the gel filtration of the aforementioned procedure. The Fab'-DOX-LP fraction was eluted in the fractions of the fraction Nos. 14 and 15, and the amount of unreacted Fab' eluted in the fraction Nos. 29 to 35 decreased compared with that observed in Example 2.

Example 4: Preparation of anti-MT1-MMP monoclonal antibody-binding liposomes

(Preparation Examples ⑨ and ⑩)

Fab'-LP and Fab'-DOX-LP were obtained in the same method as that in Example 2 except that the maleinimide molar ratio of the Fab' fraction and the maleinimide group-introduced liposomes was adjusted to 1:0.25, 1:1.6, 1:2 and 1:4.5. Fab'-LP (dilution ratio was 6.0% as measured by using HSPC as index) as Preparation Example ⑨ was prepared from LP-mal of Formulation 2 mentioned in Table 1 with a maleinimide molar ratio of 1:1.6, and Fab'-DOX-LP (dilution ratio was 21% as measured by using DOX as index) as Preparation Example ⑩ was prepared from DOX-LP-mal of Formulation 4 mentioned in Table 1 with a maleinimide molar ratio of 1:2.

Test Example 1: Confirmation of binding of anti-MT1-MMP monoclonal antibodies to liposomes

LP-mal of Formulation 2 mentioned in Table 1, DOX-LP-mal of Formulation 4 mentioned in Table 1, Fab'-DOX-LP (Preparation Examples ②, ③ and ⑩) and Fab'-LP (Preparation Example ⑥, ⑦ and ⑨) produced in Examples 2 and 4 were diluted with 6 x SDS-PAGE sample buffer (reduction) so that the phospholipid concentration was about 3 μg/lane, left for 5 minutes at 95°C, and then subjected to SDS-PAGE ("Multigel 4/20", Daiichi Pure Chemicals). In Lanes 1 to 6 where Fab'-DOX-LP or Fab'-LP was loaded, a Fab' band of about 30 kDa was observed. The above band was not observed in Lanes 7 and 8 where LP-mal or DOX-LP-mal was loaded. It was confirmed that the anti-MT1-MMP monoclonal antibodies bound to all of the liposomes bound with anti-MT1-MMP monoclonal antibody (Fig. 6).

Test Example 2: Evaluation of anti-MT1-MMP monoclonal antibody-binding liposomes for in vitro cell adhesion

1) Cytostatic test

A medium, DMEM (SIGMA), was added with potassium penicillin G (SIGMA) and streptomycin sulfate (SIGMA) at concentrations of 50 U/mL and 50 μg/mL, respectively, and further added with inactivated fetal bovine serum (Gibco) at a concentration of 10% (v/v). Subconfluent human fibrosarcoma HT1080 cells or human breast carcinoma MCF-7 cells were washed twice with 0.5 mM EDTA/PBS, adopted to residual small amount of 0.5 mM EDTA/PBS, and then left standing for about 5 minutes for separation. The cells were suspended in the medium added in an appropriate amount, and the suspension was centrifuged at room temperature at a rate of 1000 rpm for 3 minutes. After the supernatant was aspirated, a part of the suspension in which the cells were suspended in 1 to 2 mL of the medium was added with an equal volume of a trypan blue solution, thereby stained, and then counted by using a blood cell counter plate. The suspension was diluted by adding the medium to obtain a required cell density.

This cell suspension was added to a 96-well microplate in a volume of 50 μL/well, and the cells were cultured at 37°C in a $CO_2$ incubator for about 24 hours to allow the cells to adhere to the plate. Separately, DOX-LP (Table 1, Formulation 3) and Fab'-DOX-LP (Preparation Examples ② and ③) were diluted with the medium so as to obtain a phospholipid concentration required. Each of these samples was added to the cells mentioned above in a volume of 50 μL/well, and the cells were further cultured for 1 hour. In order to remove unreacted sample, the medium was removed by aspiration, and then 200 μL/well of PBS was added to wash the cells. The washing operation was repeated twice. Immediately after the washing, 100 μL/well of fresh medium was added, and the cells were further cultured for 24 hours and used for the following cell counting assay. For a part of the plates (for confirmation of the start value), the culture of 24 hours after the washing was not performed, and the cell counting assay was performed immediately after the addition of the medium.

Cell counting assay: A WST-1 solution prepared according to the instruction attached to "Cell Counting Kit" (Wako Pure Chemical Industries) and sterilized by filtration through a filter was added in a volume of 10 μL/well and stirred, and then the cells were further cultured for 4 hours. Then, A450 was measured. This A450 increases in proportion to the viable cell number.

As test groups, a blank group (only medium), control group (medium was added to the cells) and each sample group (DOX-LP or Fab'-DOX-LP was added to the cells) were prepared, and each group was examined quadruplicate (n = 4). As the phospholipid concentration (Lipid concn. (μg/mL)) of each sample, the concentration after adding a sample to the cells is indicated. The cell proliferation inhibitory rate (Inhibition) was calculated by assigning the average of A450 for each test group to the following equation.

$$\text{Inhibition} = 1 - \{(\text{Sample at 24 hr} - \text{Blank at 24 hr}) - (\text{Control at start} - \text{Blank at start})\}/\{(\text{Control at 24 hr} - \text{Blank at 24 hr}) - (\text{Control at start} - \text{Blank at start})\} \, (\%)$$

As for significance test, it was confirmed by the Bartlett's homoscedastic test that homoscedasticity was observed in each group, and then a Tukey type multiple comparison test was performed to determine presence or absence of significant difference between the DOX-LP group and the Fab'-DOX-LP group.

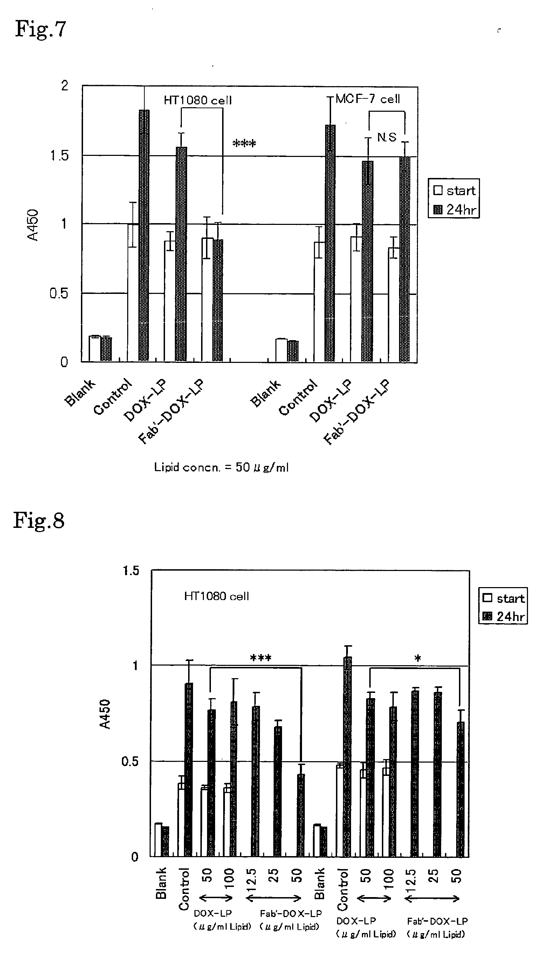

No influence of DOX-LP or Fab'-DOX-LP was observed on the start value (Figs. 7 and 8).

As for HT1080 cells, the Fab'-DOX-LP group gave a significantly lower absorbance compared with the DOX-LP group after the washing of the cells and culture for 24 hours, By the binding of the anti-MT1-MMP monoclonal antibodies, proliferation of the cells was found to be more strongly suppressed (Fig. 7), and the cell proliferation suppressing action was revealed to be dose-dependent (Fig. 8). Further, when the MCF-7 cells not expressing MT1-MMP were used, no remarkable difference was observed between the groups with and without the binding of the antibodies (Figs. 7 and 8). It was confirmed that only the anticancer agent-encapsulating liposomes bound with the anti-MT1-MMP monoclonal antibodies suppressed proliferation of the HT1080 cells expressing MT1-MMP in a dose-dependent manner.

## Supplementary explanation of Fig. 7

| Lipid concn. ($\mu$g/ml) | | Inhibition(%) | |
|---|---|---|---|
| | | HT1080 | MCF-7 |
| Control(start) | | 100% | 100% |
| Control(24hr) | | 0% | 0% |
| DOX-LP | 50 | 33% | 31% |
| Fab'-DOX-LP | 50 | 112% | 26% |

Mean±S.D.(n=4)

\*\*\* : P<0.001 by Tukey type multiple-comparison test

## Supplementary explanation of Fig. 8

| Lipid concn. ($\mu$g/ml) | | Inhibition(%) | |
|---|---|---|---|
| | | HT1080 | MCF-7 |
| Control(start) | | 100% | 100% |
| Control(24hr) | | 0% | 0% |
| DOX-LP | 50 | 26% | 37% |
| | 100 | 18% | 44% |
| Fab'-DOX-LP | 12.5 | 23% | 31% |
| | 25 | 42% | 31% |
| | 50 | 88% | 58% |

Mean±S.D.(n=4)

\*, \*\*\* : P<0.05, 0.001, respectively, by Tukey type multiple-comparison test

2) Fluorescent antibody technique

A cell suspension containing about 1.5 x $10^5$ cells/mL of the HT1080 cells, subcultured in the same manner as that in the test of 1) mentioned above, was added to a chamber slide (NUNC) in a volume of 1 mL/well, and the cells were cultured overnight. After the culture supernatant was removed by aspiration, the chamber and the slide were separated, and the slide was put into a washing bottle filled with PBS and tapped 7 times to remove non-adhering cells. This slide was left standing in a wet box and added with LP (obtained by diluting Formulation 1 mentioned in Table 1 with PBS so that the phospholipid concentration became that of Preparation Example ⑦) or Fab'-LP (Preparation Example ⑦) in a volume of 20 $\mu$L/well, and the reaction was continued in a low temperature chamber for about 1 hour under light shielding. After the reaction, the slide was washed with PBS (15 times of tapping) to remove unreacted liposome sample, immediately observed under an epi-illumination fluorescence microscope (Olympus) and photographed with a cooled CCD camera (KEYENCE). When Fab'-LP was used as the liposome sample, intense green fluorescence was observed for almost all of the cells (mainly at cell membranes). When LP not binding the antibodies was used, fluorescence was not observed. It was confirmed that only the liposomes modified with the anti-MT1-MMP monoclonal antibodies bound on the cell membranes of HT1080 cells expressing MT1-MMP.

Test Example 3: Evaluation of anti-MT1-MMP monoclonal antibody-binding liposomes for in vivo cell adhesion property (peritoneum inoculation model)

1) In vivo cell adhesion test

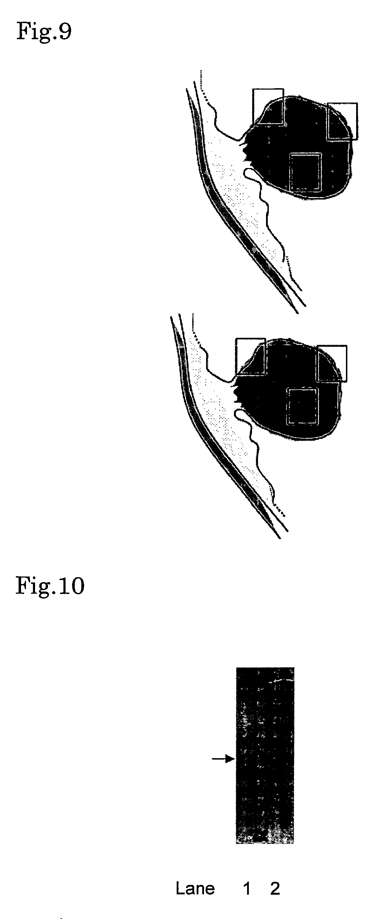

Balb-c nu/nu mice (female, 6-week old) were intraperitoneally administered with 1 x $10^6$ cells/mouse of the HT1080 cells, and then intraperitoneally administered with 50 $\mu$L/mouse of LP (Formulation 1 mentioned in Table 1 diluted with PBS so that the phospholipid concentration was the same as that of Preparation Example ⑤) or Fab'-LP (Preparation Example ⑤) on the 14th day. After 2 days, peritoneal tumor was extracted and the cleaved surface was observed under a fluorescence microscope equipped with a cooled CCD camera. On the tumor surface layer, adhesion of the liposomes (fluorescence signal) was observed in both the LP- and Fab'-LP-administered mice. In the inside of the tumor, adhesion of the liposomes (fluorescence signal) was observed only in the Fab'-LP-administered mice (Fig. 9).

2) In vivo cytotoxicity test

Balb-c nu/nu mice (female, 6-week old) were intraperitoneally administered with 1 x $10^6$ cells/mouse of the HT1080 cells, and then intraperitoneally administered with 50 $\mu$L/mouse of DOX-LP (Formulation 3 mentioned in Table 1 diluted with PBS so that the phospholipid concentration was the same as that of Preparation Example ④) or Fab'-DOX-LP (Preparation Example ④) on the 21st day. As a control, PBS was used instead of the liposomes. After 7 days, peritoneal tumor was extracted and the cleaved surface was observed by visual inspection. Further, the pathological image thereof was observed by hematoxylin/eosin (HE) staining. The HE staining was performed as follows in a conventional manner. The tumor was fixed with formalin and then embedded in paraffin, and a section sliced by using a microtome was deparaffinized with xylene (65°C, 5 minutes, immersed 3 times), dehydrated with a series of alcohol treatments (immersed 3 times in 100% ethanol for 5 minutes, and then immersed in 95% ethanol for 5 minutes), then immersed in a hematoxylin solution for 2 to 5 minutes, washed with tap water for 5 to 10 minutes to develop the color, then immersed in 95% ethanol, and immersed in an eosin solution for 10 to 30 seconds. After the staining, the section was dehydrated by a series of alcohol treatments (immersed 3 times in 100% ethanol for 5 minutes), then cleaned with xylene (immersed 3 times for 5 minutes) and mounted to prepare a HE-stained sample.

In the visual observation of the tumor surface layer and cleaved face, hemorrhagic necrosis portions were more widely observed in the surface layers of solid tumors in the DOX-LP- or Fab'-DOX-LP-administered mice compared with the control. In the Fab'-DOX-LP-administered mice, ecchymoses dispersed also inside the tumors. In the pathological images of the tumors obtained by the HE staining, solid and medullary tumor cells with vessels were observed for the control. Although hemorrhagic necrosis portions were observed in the surface layer in the DOX-LP-administered mice, deep tumor tissues were not different from those of the control and formed normal tumor tissues. Whilst in the Fab'-DOX-LP-administered mice, visual inspection revealed that uneven irregularities on the tumor surface was more remarkable compared with that of the tumor of the DOX-LP-administered mice, and the tumor tissues themselves were fragile. Furthermore, from the pathological viewpoint, the necrosis lesions following the surface layer spread into deep portions, and macular necrosis portions dispersed inside the deep tumor. Example 4: Evaluation of anti-MT1-MMP monoclonal antibody-binding liposomes for in vivo cell adhesion property (subcutaneous tumor model)

1) In vivo cytotoxicity test

Balb-c nu/nu mice (female, 6-week old) were subcutaneously administered with 1 x $10^6$ cells/mouse of the HT1080 cells on their back at two sites on the left and right, then formation of tumor was confirmed at the administration site (2 site on the left and right), and the mice were administered subcutaneously at the tumor formation site (right) or intravenously into the caudal vein with 25 μL /mouse of DOX-LP (Formulation 3 mentioned in Table 1 diluted with PBS so that the phospholipid concentration was the same as that of Preparation Example ⑧) or Fab'-DOX-LP (Preparation Example ⑧) on the 10th day. It was assumed that the tumor on the right side was a tumor reflecting the effect of the liposomes subcutaneously (locally) administered, and the tumor on the left side was a tumor reflecting the effect of the liposomes administered into the caudal vein (systemic). LP (obtained by diluting Formulation 1 mentioned in Table 1 with PBS to a phospholipid concentration of 0.46 mg/mL) was used as a control.

After 7 days, subcutaneous tumor was extracted, and the cleaved surface was observed by visual inspection. Further, the pathological image thereof and angiogenesis were observed by immunostaining using rat-derived anti-mouse CD31 monoclonal antibodies (Pharmingen, Cat. No: 557355) (counterstaining: hematoxylin staining). The immunostaining was performed as follows. A frozen section having a thickness of 8 to 10 μm was prepared in a cryostat. This frozen section is fixed with cold acetone for 10 minutes, washed with PBS and then immersed in methanol containing 0.3% $H_2O_2$ to inactivate the peroxidase activity in the tissue. This section was blocked (immersed in PBS containing 0.1% BSA (bovine serum albumin) for 20 minutes), and added dropwise with anti-CD31 antibodies diluted 100 times, and the antigen-antibody reaction was performed in a wet box for 2 hours. After the reaction, the section was washed with PBS (10 minutes x 3 times) to remove unreacted anti-CD31 antibodies, and added dropwise with HRP-labeled anti-rat antibodies (Amersham) diluted 200 times, and the antigen-antibody reaction was continued in a wet box for 30 minutes. After the reaction, the section was washed with 0.1 M PBS (10 minute x twice) to remove unreacted secondary antibodies and immersed in a phosphate buffer (pH 6.4) for about 10 minutes, and a color development reaction was performed with DAB (3,3'-diaminobenzidine tetrahydrochloride) for about 10 to 20 minutes. After the color development with DAB, the section was subjected to counterstaining with hematoxylin and mounted to prepare a CD31-stained specimen.

In the visual inspection of the cleaved surface of the tumor, ulcer was observed in the central portion of the tumor in the mice administered with Fab'-DOX-LP when compared with the LP- or DOX-LP- administered mice. Ulcer was also observed in the tumor (left) reflecting the administration into the caudal vein, and the ulcer was more remarkable in the tumor (right) reflecting the subcutaneous administration. In the pathological findings of the tumor reflecting the administration into the caudal vein, a higher anti-MT1-MMP monoclonal antibody-specific antitumor effect (necrosis of the central portion) was observed in the mice administered with Fab'-DOX-LP compared with the DOX-LP-administered mice, and disorder of the run and formation of neoplastic vessels in the tumor were observed from the results of the CD31 staining. Furthermore, in contrast to the tumor reflecting the administration of DOX-LP into the caudal vein, in which the run of vessels was rather maintained, the run of vessels was scarce in the tumor reflecting the Fab'-DOX-LP administration into the caudal vein, and thus it was suggested that the damage of neoplastic vessels by Fab'-DOX-LP might arise antecedently.

The meanings of the abbreviations used in Example 5 and the following examples are shown below.

Fab'(222-1D8)-DOX-LP : Liposomes bound with anti-MT1-MMP monoclonal antibody (clone number: 222-1D8) and encapsulating anticancer agent

Fab'(222-1D8)-NBD-LP: Liposomes bound with anti-MT1-MMP monoclonal antibody (clone number: 222-1D8) and encapsulating fluorescent agent

Fab'(222-2D12)-NBD-LP: Liposomes bound with anti-MT1-MMP monoclonal antibody (clone number: 222-2D12) and encapsulating fluorescent agent

DOX-LP: Liposomes encapsulating anticancer agent (liposomes not introduced with maleinimide group)

NBD-LP: Liposomes encapsulating fluorescent agent (liposomes introduced with maleinimide group)

DOX-LP-mal: Liposomes introduced with maleinimide group and encapsulating anticancer agent

NBD-LP-mal: Liposomes introduced with maleinimide group and encapsulating fluorescent agent

Example 5: Preparation of anti-MT1-MMP monoclonal antibody (222-1D8)-binding liposomes having various antibody binding ratios (Preparation Example ⑪ to ⑯)

A Fab' fraction (referred to as "a"), obtained in the same manner as in Example 2-1) and 2) by using anti-MT1-MMP monoclonal antibody-producing hybridoma cell (clone number: 222-1D8) obtained according to the method described in WO02/041000A1, was mixed with each liposomes (DOX-LP-mal) introduced with each of the various maleinimide groups and encapsulating the anticancer agent (doxorubicin (DOX)), i.e., Formulations 5 to 10 mentioned in Table 2 (maleinimide concentration: 0, 2.6, 5.2, 26, 52 and 104 nmol/mL, PEG-mal/PEG ratio: 0, 0.5, 1, 5, 10 and 20%, each referred to as "b") obtained in the same manner as in Example 1-1), except that NBD-C$_6$-HPC was not added, and DSPE-PEG and DSPE-PEG-MAL were added as a solution, so that the ratio of "a" and "b" was 1:1 in terms of maleinimide molar ratio.

Table 2: Formulation Examples

| Formulation | Lipid | | | DOX concentration (mg/mL) |
| --- | --- | --- | --- | --- |
| | DSPE-PEG-mal/ | Lipid composition (mM) | Total concentration (mM) | |
| 5 | 0 | HSPC/Chol/DSPE-PEG (11.59/7.89/0.534) | 20.01 | 2 |
| 6 | 0.5 | HSPC/Chol/DSPE-PEG/DSPE-PEG-mal (11.59/7.89/0.531/0.003) | 20.01 | 2 |
| 7 | 1 | HSPC/Chol/DSPE-PEG/DSPE-PEG-mal (11.59/7.89/0.529/0.005) | 20.01 | 2 |
| 8 | 5 | HSPC/Chol/DSPE-PEG/DSPE-PEG-mal (11.59/7.89/0.507/0.027) | 20.01 | 2 |
| 9 | 10 | HSPC/Chol/DSPE-PEG/DSPE-PEG-mal (11.59/7.89/0.481/0.053) | 20.01 | 2 |
| 10 | 20 | HSPC/Chol/DSPE-PEG/DSPE-PEG-mal (11.59/7.89/0.427/0.107) | 20.01 | 2 |
| 11 | 10 | HSPC/Chol/DSPE-PEG/DSPE-PEG-mal (28.20/19.20/1.17/0.13) | 48.70 | 0 |
| 12 | 0 | HSPC/Chol/DSPE-PEG/DSPE-PEG-mal (28.20/19.20/1.30/0) | 48.70 | 0 |

After the reaction was performed for 20 hours in a low temperature chamber under light shielding, unreacted thiol groups were blocked with N-ethylmaleinimide (0.1 M aqueous solution was added) in a molar amount of 10 times the amount of Fab'. This reaction mixture was fractioned by using a Sepharose CL-4B column (diameter: 1.5 or 3.0 cm x length: 47 cm) equilibrated with PBS into 2- or 8-mL fractions, and anti-MT1-MMP monoclonal antibody (clone number 222-1D8)-binding and anticancer agent-encapsulating liposomes (Fab'(222-1D8)-DOX-LP) fractions were collected from the void volume and pooled in the same manner as in Example 2-3) to obtain Fab'-(222-1D8)-DOX-LP (Preparation Examples ⑪ to ⑯). Unreacted Fab' was eluted around the fraction Nos. 29 to 35, and thus it was confirmed that the liposome fraction and unreacted Fab' were separated by the gel filtration.

As for the lipid concentration of the liposomes or anti-MT1-MMP monoclonal antibody-binding liposomes, the cholesterol concentrations measured by using Cholesterol E-Test Wako (Wako Pure Chemical Industries) were used as the lipid concentrations. In addition, no influence of DOX per se was observed on the measurement system, and favorable correlation was observed between the cholesterol concentration and DOX concentration measured by HPLC. Therefore, in the following test examples, the liposomes were diluted with PBS for use so that the liposomes not binding antibodies and the liposomes binding the antibodies had the same cholesterol concentration.

Example 6: Preparation of anti-MT1-MMP monoclonal antibody (222-2D12)-binding liposomes (Preparation Example ⑰) and control liposomes (Preparation Examples ⑱ and ⑲)

Fab'(222-2D12)-NBD-LP (Preparation Example ⑰) was obtained from NBD-LP-mal (maleinimide concentration: 130 nmol/mL, PEG-mal/PEG ratio: 10%) of Formulation 11 mentioned in Table 2 added with NBD-$C_6$-HPC and not encapsulating DOX in the same manner as in Example 5, except that a Fab' fraction obtained in the same manner as in Example 2-1) and 2) by using anti-MT1-MMP monoclonal antibody-producing hybridoma cells of the clone number 222-2D12 obtained according to the method described in WO02/041000 was used, and the maleinimide molar ratio of the maleinimide group-introduced liposomes was adjusted to 1:3. Further, as control examples for the above liposomes, Fab'(222-1D8)-NBD-LP (Preparation Example ⑱) comprising the same NBD-LP-mal binding Fab' derived from the antibodies of the clone number 222-1D8 and liposomes of NBD-LP of Formulation 12 mentioned in Table 2 subjected to gel filtration (Preparation Example ⑲) were similarly prepared.

Test Example 5: Confirmation of binding of anti-MT1-MMP antibodies to liposomes

1) Confirmation based on competition with HRP-Fab'

Human MT1-MMP (150 μg/mL) purified from recombinant *Escherichia coli* was diluted 6000 times with 0.1 M Na-P pH 7.0 and sufficiently stirred. The cells were added to an immunomodule set on a 96-well plate frame in a volume of 100 μL/well, and after the plate was sealed, left standing in a low temperature chamber more than one night to coat the antigen (referred to as "a").

Each of the various liposomes prepared in Examples 5 and 6 (Preparation Example ⑪ to ⑲) was diluted with PBS so that the cholesterol concentration was 10 μg/mL, added with the same volume of phosphate buffer containing 0.4% Tween 20, mixed and then left standing overnight in an incubator at 37°C to perform a treatment with surfactant (referred to as "b"). The sample "a" was washed 3 times with a phosphate buffer containing 0.1% Tween 20 in a volume of 300 μL/well, added with 300 μL/well of 10 mM IRB (1% BSA, 10 mM EDTA · 2Na, 30 mM $Na_2HPO_4$· $12H_2O$, 0.1 M NaCl) and left standing in an incubator at 25°C for 1 hour for blocking (referred to as "c"). IgG (222-1D8) for standard curve was diluted with PBS to a concentration of 100 μg/mL and further serially diluted with a phosphate buffer containing 0.2% Tween 20 to prepare serially diluted solutions for standard curve (12.5, 3.125, 0.781, 0.195, 0.049 and 0 μg/mL) (referred to as "d"). HRP-Fab' (222-1D8-derived Fab' labeled with horse radish peroxidase) was diluted with 10 mM IRB to prepare a 0.125 μg/mL solution (referred to as e). The sample "b" or "d" and the sample "e" were mixed at a volume ratio of 1:4 (referred to as "f"). The sample "c" was washed 3 times with 300 μL/well of phosphate buffer, then added with 100 μL/well (n = 2) of "f", and then left standing in an incubator at 25°C for 1 hour to perform the antigen-antibody reaction (competitive reaction) (referred to as "g"). The sample "g" was washed 3 times with 300 μL/well of phosphate buffer containing 0.1% Tween 20, then added with 100 μL/well of TMB (Bio FX Laboratories), and then left standing in an incubator at 25°C for 15 minutes to perform an enzymatic reaction of HRP with TMB as a substrate (referred to as "h"). The sample "h" was added with 100 μL/well of 1 N aqueous $H_2SO_4$ to terminate the reaction, and A450 was measured immediately. In addition, a well for 0 μg/mL was prepared in the sample "d", and a well not coated with the antigen was prepared in the sample "a", which were used as control and blank, respectively. From A450 of the series for standard curve, it was confirmed that the reaction was a IgG concentration-dependent competitive reaction (Table 3).

Table 3: Evaluation of binding of antibodies to liposomes (creation of standard curve)

| | | A450 | | | Competition ratio (%) |
|---|---|---|---|---|---|
| | | Measured value 1 | Measured value 2 | Average | |
| Blank | | 0.009 | 0.009 | 0.009 | 100% |
| Control | | 2.115 | 2.152 | 2.134 | 0% |
| Final concentration of IgG for standard curve (μg/mL) | 2.500 | 0.139 | 0.147 | 0.143 | 94% |
| | 0.625 | 0.341 | 0.334 | 0.338 | 85% |
| | 0.156 | 0.683 | 0.751 | 0.717 | 67% |
| | 0.039 | 1.379 | 1.237 | 1.308 | 39% |
| | 0.010 | 1.877 | 1.792 | 1.835 | 14% |

This IgG concentration-dependent competition curve was used as a standard curve to calculate the amount in terms of IgG in each antibody liposome sample. The absorbance observed with Fab'(222-1D8)-DOX-LP or Fab'(222-1D8)-NBD-LP used as the sample was apparently lower than that observed with the solvent used as the sample (control), or with liposomes not binding antibody or the 222-2D12 antibody-binding liposomes as the sample (non-competitive specimen), and thus the antigen-antibody reaction of HRP-Fab'was competed. In Preparation Example ⑪ to ⑯ prepared by binding the liposomes having maleinimide groups at various concentrations with the antibodies, competition ratios were increased in proportion to the maleinimide concentration. Specifically, the calculated amount in terms of IgG per unit amount of cholesterol changed substantially in proportion to the maleinimide concentration of used DOX-LP-mal, and thus it was confirmed that Fab'(222-1D8)-DOX-LP was obtained with various antibody-binding ratios (Table 4).

Table 4: Evaluation of binding of antibodies to liposomes

| | | A450 | | | Competition ratio (%) | Amount in terms of IgG | |
|---|---|---|---|---|---|---|---|
| | | | | | | IgG conc.a) | IgG/Choll.b) |
| | | Measured value 1 | Measured value 2 | Average | | (μg/mL) | (μg/mg) |
| Preparation Example (PEG·mal/PEG ratio in starting formulation) | ⑪ (0%) | 2.067 | 2.114 | 2.091 | 2% | 2.6 | 6 |
| | ⑫ (0.5%) | 1.578 | 1.648 | 1.613 | 24% | 6.9 | 17 |
| | ⑬ (1%) | 1.360 | 1.402 | 1.381 | 35% | 13.5 | 30 |
| | ⑭ (5%) | 1.107 | 1.122 | 1.115 | 48% | 25.7 | 58 |
| | ⑮ (10%) | 0.679 | 0.746 | 0.713 | 67% | 74.0 | 172 |
| | ⑯ (20%) | 0.465 | 0.486 | 0.476 | 78% | 145.4 | 356 |

a): IgG concentration,　b): IgG amount per cholesterol

From the above, for controlling the antibody binding ratio of the antibody-binding liposomes, it was found that a change in the amount of maleinimide introduced into the liposomes at the time of the preparation of the antibody-binding liposomes was effective.

2) Confirmation by SDS-PAGE

Fab'(222-2D12)-NBD-LP (Preparation Example ⑰) was diluted with 6 x SDS-PAGE sample buffer (reduction) so that the cholesterol concentration became about 0.8 μg/lane, and F(ab')$_2$ derived from the antibody of the clone number 222-2D12 was diluted with 6 x SDS-PAGE sample buffer (reduction) so that the protein concentration was about 1 μg/lane. The both samples were left at 95°C for 5 minutes, then loaded on 15% SDS-PAGE gel and stained with CBB.

In both of Lane 1 (Fab' (222-2D12)-NBD-LP (Preparation Example (17))) and Lane 2 (F(ab')$_2$ derived from 222-2D12 antibody), a Fab' band of about 30 kDa was observed. Therefore, the antibodies were found to bind to the anti-MT1-MMP monoclonal antibody (clone number: 222-2D12)-binding liposomes of Preparation Example ⑰ (Fig. 10).

Test Example 6: Evaluation of anti-MT1-MMP monoclonal antibody-binding liposomes for in vitro cell adhesion property

1) Cytostatic test

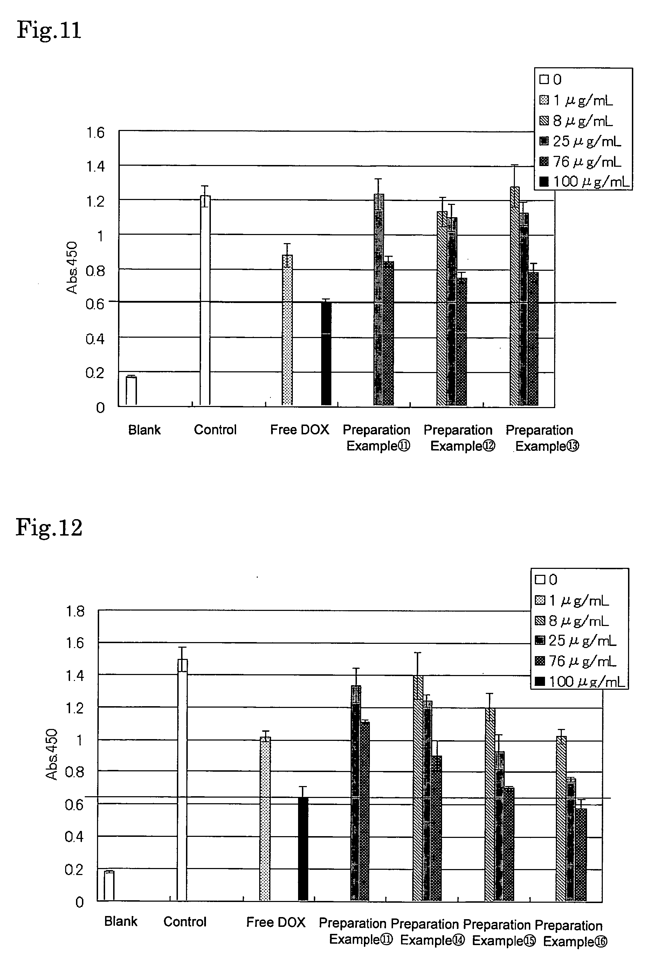

According to the method described in Test Example 2-1), the cytostatic ability of various antibody-binding liposomes was evaluated by using the HT1080 cells. The cells were adhered to a 96-well plate, then added with anti-MT1-MMP monoclonal antibody (clone number: 222-1D8)-binding and anticancer agent-encapsulating liposomes (Fab'(222-1D8)-DOX-LP), and cultured for 1 hour. After the culture, the cells were washed, added with a fresh medium, and further cultured for 24 hours. After the cells were washed and cultured for 24 hours, a cell counting assay was performed, and A450 serving as an index of viable cell count was plotted. Means ± S.D. (n = 4) was indicated for each group. As control, only the medium (control), anticancer agent-encapsulating liposomes not bound with antibody (DOX-LP (Preparation Example ⑪) or the anticancer agent alone (free DOX) was added instead of Fab'(222-1D8)-DOX-LP. A group in which only the medium was similarly treated without adding the cells was used as a blank. It was confirmed by multiple times of experiments that the start value used in the calculation of inhibitory rate and the like in Test Example 2 was not substantially different from the absorbance obtained with 100 μg/mL of DOX alone (free DOX) used as the sample, and therefore the value obtained for the 100 μg/mL free DOX group was used as the start value. This start value is indicated around the A450 value of 0.6 in Figs. 11 and 12.

As a result of experiments for Preparation Examples ⑪ to ⑯, almost no cytostatic activity was observed for Preparation Examples ⑫ and ⑬, which were prepared from DOX-LP-mal having a PEG-mal/PEG ratio of less than 1% (Formulations 6 and 7 mentioned in Table 2). Whilst cytostatic activity was observed for Preparation Examples ⑭ to ⑯, which were prepared from DOX-LP-mal having a PEG-mal/PEG ratio of 5% or more (Formulations 8 to 10 mentioned in Table 2), and the activities thereof were proportional to the PEG-mal/PEG ratio of the starting materials. Specifically, it was demonstrated that Preparation Example ⑰ prepared from DOX-LP-mal having the ratio of 20% (Formulation 10 mentioned in Table 2) gave a cytostatic activity about 10 times that of DOX-LP as the control, Preparation Example ⑮ prepared from DOX-LP-mal having the ratio of 10% (Formulation 9 mentioned in Table 2) gave a cytostatic activity about 6 to 9 times that of the control, and Preparation Example ⑭ prepared from DOX-LP-mal having the ratio of 5% (Formulation 8 mentioned in Table 2) gave a cytostatic activity about 2 times that of the control (Figs. 11 and 12). In Table 5, cytostatic ratios for the HT1080 cells at a DOX concentration of 25 μg/mL (100 - [Sample]/([Control] - [DOX (100 μg/mL)]) x 100, the values in [ ] are averages of A450 values for each group) are summarized.

Table 5: Cytostatic ratio for HT1080 cells at DOX concentration of 25 $\mu$ g/mL

| | Control group | Liposome |
|---|---|---|
| Control | 0% | |
| Free DOX (100 $\mu$ g/mL) | 100% | |
| Preparation Example ⑪ (DOX-LP, 0%*) | | 8%** |
| Preparation Example ⑫ (0.5%*) | | 19% |
| Preparation Example ⑬ (1%*) | | 15% |
| Preparation Example ⑭ (5%*) | | 30% |
| Preparation Example ⑮ (10%*) | | 67% |
| Preparation Example ⑯ (20%*) | | 87% |

\* PEG-mal/PEG ratio,

\*\* Average of results of 2 of experiments (Figs. 11 and 12)

From the result of Test Example 5-1) and the result of Test Example 6-1) mentioned above, it was suggested that the maleinimide introduction rate of the maleinimide-introduced liposomes used for binding of antibodies, the presumed antibody binding ratio of the antibody-binding liposomes obtained by the competitive method and the HT1080 cell cytostatic ability are in a parallel relation.

These examples and test examples are for those demonstrating preparation of antibody-binding liposomes having various antibody binding ratios, for demonstrating the method for evaluating the cytostatic ability of the antibody-binding liposomes having various antibody binding ratios, and those for demonstrating that the amount of antibodies binding to the liposomes is one of the factors for controlling cytostatic ability of the antibody-binding liposomes, and these examples do not limit formulations, preparation methods, numbers of maleinimide groups, types of antibody, antibody binding ratios, types and concentrations of a medicament to be encapsulated and the like of the liposomes of the present invention bound with the antibody.

2) Fluorescent antibody technique

Fluorescent antibody staining was performed according to the method described in Test Example 2-2) by using HT1080 cells fixed with PLP (periodate-lysine-paraformaldehyde).

The test was performed by using solutions obtained by diluting Preparation Examples ⑰ to ⑲ with PBS so that the cholesterol concentration became about 100 $\mu$ g/mL as samples. In addition, it was confirmed that the fluorescence intensities of these diluted sample solutions were substantially the same by fluorescence intensity measurement using a fluorescence absorbance plate reader (Perkin-Elmer, Wallac 1420 Multi-label Counter).

When Fab' (222-2D12)-NBD-LP (Preparation Example ⑰) or Fab' (222-1D8)-NBD-LP (Preparation Example ⑱ ) was reacted with the HT1080 cells, green fluorescence was observed for almost all cells (mainly at cell membranes). Whilst fluorescence was not observed for NBD-LP not bound with these antibodies (Preparation Example ⑲).

It was confirmed that only the liposomes bound with the anti-MT1-MMP antibodies adhered to cell membranes of HT1080 cells expressing MT1-MMP, and this adhesion was similarly observed for the liposomes bound with anti-MT1-MMP monoclonal antibody 222-1D8 and 222-2D12.

Test Example 7: Evaluation of anti-MT1-MMP monoclonal antibody-binding liposomes for in vivo cell adhesion property (subcutaneous tumor model)

1) In vivo liposome uptake test

Balb-c nu/nu mice (female, 7- to 8-week old) were subcutaneously transplanted with 1 x 10⁶ cells/mouse of the HT1080 cells on their back and then administered with 15 $\mu$ g (amount of cholesterol)/200 $\mu$ L/mouse of NBD-LP (Preparation Example ⑲, Fab'(222-1D8)-NBD-LP (Preparation Example ⑱ ) or Fab'(222-2D12)-NBD-LP (Preparation Example ⑰) diluted with PBS on the 14th to 21st days into the caudal vein. Two hours after the administration, the subcutaneous tumor was extracted, and a tissue slice having a thickness of about 2 to 3 mm was prepared with sharp scissors, lightly washed with PBS for 30 minutes and observed with a fluorescence microscope with cooled CCD camera.

In the Fab'(222-1D8)-NBD-LP (Preparation Example ⑱ and Fab' (222-2D12)-NBD-LP (Preparation Example ⑰) administration groups, a specific fluorescence signal was detected especially around circumference vessels (neoplastic vessels) of a tumor having a small diameter (less than 1 cm). For the liposomes not bound with antibody (Preparation Example ⑲, NBD-LP), no clear fluorescence signal was observed in tumor tissues including circumference vessels (neoplastic vessels). From the results mentioned above, it was demonstrated that the anti-MT1-MMP monoclonal antibody-binding liposomes were significantly accumulated in the circumference vessels (neoplastic vessels) of proliferating tumor.

Test Example 8: Evaluation of anti-MT1-MMP monoclonal antibody-binding liposomes for stealth property

1) Evaluation of in vitro aggregation property

A solution in a volume of 40 $\mu$ L obtained by diluting each of Preparation Examples ⑪ to ⑲ with PBS, so that the cholesterol concentration was 75 $\mu$ g/mL, and 60 $\mu$ L of PBS or a 10% FCS/DMEM medium were mixed and then the mixture was left standing at room temperature for 15, 30 or 60 minutes (only for 30 minutes for Preparation Examples ⑰ to ⑲. After the mixture was lightly stirred, absorbance (630 nm) was measured, and presence or absence of aggregations of liposomes was observed under a stereoscopic microscope. For Preparation Example ⑪ to ⑮ and ⑰ to ⑲, any difference in absorbance was not observed irrespective of the use of PBS or 10% FCS/DMEM as the solvent to be mixed, and no difference in absorbance was observed in comparison with the corresponding liposomes bound with the antibody (Preparation Examples ⑪ and ⑲). However, when Preparation Example⑯ and the 10% FCS/DMEM medium were mixed, an apparent increase of absorbance resulting from aggregation was observed, and a clear aggregation was also observed under microscopic observation (Table 6).

Table 6: Evaluation of antibody-binding liposomes for in vitro aggregation property

| Preparation Example No. | Absorbance (630 nm)* | | | | | | Microscopic observation** | |
|---|---|---|---|---|---|---|---|---|
| | 15 minutes | | 30 minutes | | 60 minutes | | | |
| | PBS | +FCS | PBS | +FCS | PBS | +FCS | PBS | +FCS |
| PBS | 0.0352 | 0.0354 | 0.0349 | 0.0355 | 0.0350 | 0.0366 | - | - |
| ⑪ (0%) | 0.0401 | 0.0397 | 0.0391 | 0.0396 | 0.0393 | 0.0407 | - | - |
| ⑫ (0.5%) | 0.0374 | 0.0389 | 0.0373 | 0.0385 | 0.0387 | 0.0397 | - | - |
| ⑬ (1%) | 0.0380 | 0.0377 | 0.0379 | 0.0376 | 0.0380 | 0.0391 | - | - |
| ⑭ (5%) | 0.0372 | 0.0387 | 0.0370 | 0.0382 | 0.0374 | 0.0389 | - | - |
| ⑮ (10%) | 0.0386 | 0.0388 | 0.0384 | 0.0389 | 0.0384 | 0.0392 | - | - |
| ⑯ (20%) | 0.0402 | 0.0634 | 0.0413 | 0.0621 | 0.0410 | 0.0597 | - | + |
| PBS | | | 0.0359 | 0.0363 | | | - | - |
| ⑰(222-2D12) | | | 0.0535 | 0.0518 | | | - | - |
| ⑱(222-1D8) | | | 0.0511 | 0.0509 | | | - | - |
| ⑲(0%) | | | 0.0499 | 0.0489 | | | - | - |

*Average for N = 2

**: - indicates absence of aggregation, and + indicates presence of aggregation.

2) In vivo blood retentivety test

Balb/cAnNCrj-nu mice (male, 6- to 7-week old) were administered with DOX-LP (Preparation Example ⑪ or Fab'(222-1D8)-DOX-LP (Preparation Examples ⑭ to ⑯ concentrated by ultracentrifugation in an amount of 7.5 mg (amount of DOX determined by HPLC measurement)/kg each into the caudal vein. Plasma was collected from each mouse 2, 6, 24, 48 and 72 hours after the administration, and the plasma concentration of non-metabolized DOX was measured by HPLC fluorescence detecting method. In Table 7, averages of DOX plasma concentrations obtained from the mice (n = 3) for each blood collection time point of each group are indicated. Substantially similar changes in the plasma concentration were obtained for Preparation Examples ⑪ , ⑭ and ⑯, whilst as for Preparation Example ⑰, the plasma concentration was quickly reduced after the administration, and then, retention of only about 1/10 of that of Preparation Examples ⑪ , ⑭ and ⑮ was observed (Table 7).

Table 7: Evaluation of antibody-binding liposomes for in vitro blood retentivety

| Preparation Example No. | Plasma DOX concentration (μg/mL) | | | | |
|---|---|---|---|---|---|
| | After 2 hours | After 6 hours | After 24 hours | After 48 hours | After 72 hours |
| (11) (0%*) | 55.2 | 35.1 | 14.3 | 5.1 | 3.5 |
| (14) (5%*) | 41.4 | 42.4 | 25.3 | 15.2 | 1.7 |
| (15) (10%*) | 30.9 | 27.9 | 10.4 | 3.6 | 2.6 |
| (16) (20%*) | 4.8 | 4.4 | 2.3 | 2.1 | 0.3 |
| *PEG-mal/PEG ratio | | | | | |

From the results described above, it was suggested that the increase in the absorbance (630 nm), observed after mixing with FCS in the in vitro aggregation evaluation test, reflected aggregation of liposomes, and the in vivo blood retentivity was clearly degraded by the aggregation. Further, from the fact that the antibody-binding liposomes, which gave aggregation due to the mixing with FCS and thus gave the decrease in the in vivo blood retentivity, were only those of Preparation Example ⑰ (prepared form DOX-LP-mal having a PEG-mal/PEG ratio of 20%), the liposomes bound with a lot of antibodies was found to reduce the stealth property. In contrast, Preparation Example ⑮ prepared from DOX-LP-mal having a PEG-mal/PEG ratio of 10% gave no reduction of the stealth property and exhibited significant cytostatic ability as shown by Test Example 6, and therefore it was suggested that the formulation of Preparation Example ⑮ was a more preferred formulation.

These examples and test examples are those for demonstrating the methods for evaluating aggregation property of the antibody-binding liposomes having various antibody binding ratios, those for demonstrating the methods for evaluating blood retentivity, and those for demonstrating that excessively antibodies loaded to the liposomes was one of the factors that degrade the performances of the liposomes bound with antibody, and these examples do not limit the formulations, preparation methods, numbers of maleinimide groups, types of antibody, antibody binding ratios, types and concentrations of a medicament to be encapsulated and the like of the liposomes of the present invention bound with the antibody.

Industrial Applicability

The lipid membrane structures containing an anti-MT-MMP monoclonal antibody of the present invention can efficiently deliver a medicinally active ingredient and/or a gene to tumor cells which express a membrane-type matrix metalloproteinase (MT-MMP), and are useful as a drug delivery system that can efficiently deliver a medicinally active ingredient and/or a gene also to an angiogenesis front inside a tumor.

**Claims**

1. A lipid membrane structure containing an anti-membrane-type matrix metalloproteinase monoclonal antibody.

2. The lipid membrane structure according to claim 1, wherein the monoclonal antibody is present in a lipid membrane, on a surface of lipid membrane, in a internal space of lipid membrane, in a lipid layer, and/or on a surface of lipid layer of the lipid membrane structure.

3. The lipid membrane structure according to claim 1, which comprises the monoclonal antibody as a component of the lipid membrane structure.

4. The lipid membrane structure according to claim 1, wherein the monoclonal antibody binds to a membrane surface of the lipid membrane structure.

5. The lipid membrane structure according to any one of claims 1 to 4, wherein the monoclonal antibody consists of one or more kinds of monoclonal antibodies selected from an anti-MT1-MMP monoclonal antibody, an anti-MT2-MMP monoclonal antibody, an anti-MT3-MMP monoclonal antibody, an anti-MT4-MMP monoclonal antibody, an anti-MT5-MMP monoclonal antibody, and an anti-MT6-MMP monoclonal antibody.

6. The lipid membrane structure according to any one of claims 1 to 5, wherein the monoclonal antibody is a human monoclonal antibody or a mouse monoclonal antibody.

7. The lipid membrane structure according to any one of claims 1 to 6, wherein the monoclonal antibody is a Fab fragment, a F(ab')$_2$ fragment, or a Fab' fragment.

8. The lipid membrane structure according to any one of claims 1 to 7, which contains a substance for binding the monoclonal antibody to the lipid membrane structure.

9. The lipid membrane structure according to claim 8, wherein the substance for binding the monoclonal antibody to the lipid membrane structure is a lipid derivative that can react with mercapto group in the anti-MT-MMP monoclonal antibody or a fragment thereof.

10. The lipid membrane structure according to any one of claims 1 to 9, which contains a phospholipid and/or a phospholipid derivative as a component of the lipid membrane structure.

11. The lipid membrane structure according to claim 10, wherein the phospholipid and/or the phospholipid derivative consists of one or more kinds of phospholipids and/or phospholipid derivatives selected from the group consisting of phosphatidylethanolamine, phosphatidylcholine, phosphatidylserine, phosphatidylinositol, phosphatidylglycerol, cardiolipin, sphingomyelin, ceramide phosphorylethanolamine, ceramide phosphorylglycerol, ceramide phosphorylglycerol phosphate, 1,2-dimyristoyl-1,2-deoxyphosphatidylcholine, plasmalogen and phosphatidic acid.

12. The lipid membrane structure according to any one of claims 1 to 11, which further contains a sterol as a component of the lipid membrane structure.

13. The lipid membrane structure according to claim 12, wherein the sterol is cholesterol and/or cholestanol.

14. The lipid membrane structure according to any one of claims 1 to 13, which has a blood retentive function.

15. The lipid membrane structure according to claim 14, which contains a blood retentive lipid derivative as a component of the lipid membrane structure.

16. The lipid membrane structure according to claim 15, wherein the blood retentive lipid derivative is a polyethylene glycol-lipid derivative or a polyglycerin-phospholipid derivative.

17. The lipid membrane structure according to claim 16, wherein the polyethylene glycol-lipid derivative consists of one or more kinds of polyethylene glycol-lipid derivatives selected from the group consisting of N-{carbonyl-methoxypolyethylene glycol-2000}-1,2-dipalmitoyl-sn-glycero-3-phosphoethanolamine, N-{carbonyl-methoxypolyethylene glycol-5000}-1,2-dipalmitoyl-sn-glycero-3-phosphoethanolamine, N-{carbonyl-methoxypolyethylene glycol-750}-1,2-distearoyl-sn-glycero-3-phosphoethanolamine, N-{carbonyl-methoxypolyethylene glycol-2000}-1,2-distearoyl-sn-glycero-3-phosphoethanolamine and N-{carbonyl-methoxypolyethylene glycol-5000}-1,2-distearoyl-sn-glycero-3-phosphoethanolamine.

18. The lipid membrane structure according to any one of claims 1 to 17, which has a temperature change-sensitive function.

19. The lipid membrane structure according to claim 18, which contains a temperature-sensitive lipid derivative as a component in the lipid membrane structure.

20. The lipid membrane structure according to claim 19, wherein the temperature-sensitive lipid derivative is dipalmitoylphosphatidylcholine.

21. The lipid membrane structure according to any one of claims 1 to 20, which has a pH-sensitive function.

22. The lipid membrane structure according to claim 21, which contains a pH-sensitive lipid derivative as a component of the lipid membrane structure.

23. The lipid membrane structure according to claim 22, wherein the pH-sensitive lipid derivative is dioleoylphosphatidylethanolamine.

24. The lipid membrane structure according to any one of claims 1 to 23, which reacts with a membrane-type matrix metalloproteinase on a tumor cell membrane.

25. The lipid membrane structure according to claim 24, wherein the tumor cell is an MT-MMP expressing cell.

26. The lipid membrane structure according to claim 24 or 25, wherein the tumor cell is a cell of fibrosarcoma, squamous carcinoma, neuroblastoma, breast carcinoma, gastric cancer, hepatoma, bladder cancer, thyroid tumor, urinary tract epithelial cancer, glioblastoma, acute myeloid leukemia, pancreatic duct cancer or prostate cancer.

**27.** The lipid membrane structure according to any one of claims 1 to 26, which reacts with a membrane-type matrix metalloproteinase of a neoplastic vessel.

**28.** The lipid membrane structure according to any one of claims 1 to 27, wherein the lipid membrane structure is in the form of micelle, emulsion, liposome or a mixture thereof.

**29.** The lipid membrane structure according to any one of claims 1 to 28, which is in a form of dispersion in an aqueous solvent, a lyophilized form, a spray-dried form or a frozen form.

**30.** A pharmaceutical composition comprising the lipid membrane structure according to any one of claims 1 to 29 and a medicinally active ingredient and/or a gene.

**31.** The pharmaceutical composition according to claim 30, wherein the medicinally active ingredient and/or gene is present in a lipid membrane, on a surface of lipid membrane, in an internal space of lipid membrane, in a lipid layer and/or on a surface of lipid layer of the lipid membrane structure.

**32.** The pharmaceutical composition according to claim 30 or 31, which is in a form of a dispersion in an aqueous solvent, a lyophilized form, a spray-dried form, or a frozen form.

**33.** A method for estimating an amount of monoclonal antibody against an anti-membrane-type matrix metalloproteinase contained in the lipid membrane structure according to any one of claims 1 to 23, wherein a competitive reaction against an antigenic substance caused by both of an enzyme-labeled monoclonal antibody, prepared from the monoclonal antibody against a membrane-type matrix metalloproteinase by a known method, and the lipid membrane structure is detected by an enzyme immunoassay technique.

Fig.1

Fig.2

Fig.3

Fig.4

Fig.5

Fig.6

Fig.7

Lipid concn. = 50 μg/ml

Fig.8

Fig.9

Fig.10

Lane   1  2

## Fig.11

## Fig.12

## INTERNATIONAL SEARCH REPORT

| International application No. |
| --- |
| PCT/JP2004/004876 |

**A. CLASSIFICATION OF SUBJECT MATTER**
Int.Cl⁷ A61K47/42, A61K47/48, A61K31/704, A61K9/127, A61K9/107,
A61K9/16, A61K9/19, A61K39/395, A61P35/00, A61P35/04

According to International Patent Classification (IPC) or to both national classification and IPC

**B. FIELDS SEARCHED**

Minimum documentation searched (classification system followed by classification symbols)
Int.Cl⁷ A61K47/42, A61K47/48, A61K31/704, A61K9/127, A61K9/107,
A61K9/16, A61K9/19, A61K39/395, A61P35/00, A61P35/04

Documentation searched other than minimum documentation to the extent that such documents are included in the fields searched

Electronic data base consulted during the international search (name of data base and, where practicable, search terms used)
CAP(STN), BIOSIS(STN), MEDLINE(STN), EMBASE(STN), WPI, JOIS

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | WO 02/72011 A2 (TARGESOME INC.),<br>19 September, 2002 (19.09.02),<br>Full text; Claim 15; page 42, lines 6 to 9;<br>Fig. 7<br>& US 2002/197210 A        & EP 1372739 A2<br>& AU 2002/245629 B | 1-33<br>1-33 |
| Y | WO 02/41000 A1 (DAIICHI FINE CMEM CO., LTD.),<br>23 May, 2002 (23.05.02),<br>Full text<br>& AU 2002/23129 B        & EP 1336851 A1<br>& US 2004/96899 A | 1-33 |

| ☒ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>01 July, 2004 (01.07.04) | Date of mailing of the international search report<br>27 July, 2004 (27.07.04) |
| --- | --- |
| Name and mailing address of the ISA/<br>Japanese Patent Office | Authorized officer |
| Facsimile No. | Telephone No. |

Form PCT/ISA/210 (second sheet) (January 2004)

INTERNATIONAL SEARCH REPORT

| | International application No. |
|---|---|
| | PCT/JP2004/004876 |

C (Continuation). DOCUMENTS CONSIDERED TO BE RELEVANT

| Category* | Citation of document, with indication, where appropriate, of the relevant passages | Relevant to claim No. |
|---|---|---|
| Y | KITAGAWA, Y. et al., "Expression and tissue localization of mambrane-types 1, 2, and 3 matrix metalloproteinases in human urothelial carcinomas." J.Urol., (1998) Vol.160, No.4, pages 1540 to 1545 | 1-33 |
| Y | YOSHIZAKI, T. et al., "Increased expression of membrane type 1-matrix metalloproteinase in head and neck carcinoma." Cancer, (1997) Vol.79, No.1, pages 39 to 44 | 1-33 |
| Y | AOKI, T. et al., "Two-step sandwich enzyme immunoassay using monoclonal antibodies for detection of soluble and mambrane-associated human mambrane typo 1-matrix metalloproteinase." J.Immunoassay Immunochem., (2002) Vol.23, No.1, pages 49 to 68 | 1-33 |
| Y | WO 00/64413 A1 (MITSUBISHI CHEM. CORP.), 02 November, 2000 (02.11.00), Full text; Claims; pages 8 to 9; examples & AU 2000/38407 B & EP 1174126 A1 | 1-33 |
| Y | WO 03/9870 A1 (MITSUBISHI PHARMA CORP.), 06 February, 2003 (06.02.03), Full text; Claims; page 8, line 23 to page 15, line 15 & EP 1410806 A1 | 1-33 |

Form PCT/ISA/210 (continuation of second sheet) (January 2004)

36